# EUROPÄISCHE PATENTSCHRIFT

(11) **EP 2 846 858 B1**
(45) Veröffentlichungstag und Bekanntmachung des Hinweises auf die Patenterteilung: **09.01.2019**
(21) Anmeldenummer: 13719555.8
(22) Anmeldetag: 30.04.2013
(51) Int. Cl.: A61M 15/00, A61M 11/02

(54) **ZERSTÄUBER**
ATOMISER
PULVÉRISATEUR

(30) Priorität: 09.05.2012 EP 12167219; 29.10.2012 EP 12190436
(43) Veröffentlichungstag der Anmeldung: 18.03.2015
(73) Patentinhaber: Boehringer Ingelheim International GmbH, 55216 Ingelheim am Rhein (DE)
(72) Erfinder: WINKLER, Robert Gerhard, 63739 Aschaffenburg (DE); WACHTEL, Herbert, 55216 Ingelheim am Rhein (DE); DUNNE, Stephen Terence, Europark Ipswich IP3 9BF (GB); JUNG, Andree, 55216 Ingelheim am Rhein (DE)
(74) Vertreter: Simon, Elke Anna Maria
(86) Internationale Anmeldenummer: PCT/EP2013/059011
(87) Internationale Veröffentlichungsnummer: WO 2013/167429

(56) Entgegenhaltungen:
- EP-A1- 1 731 186
- EP-A1- 2 042 208
- WO-A1-02/056948
- WO-A1-2004/110536
- WO-A1-2011/067763
- WO-A2-03/045483
- WO-A2-2006/090149
- WO-A2-2009/040044
- WO-A2-2011/077414
- GB-A- 1 562 098
- US-A1- 2002 088 462
- US-A1- 2011 297 151
- US-B1- 7 533 668

## Beschreibung

Die vorliegende Erfindung betrifft Geräte zur Zerstäubung von medizinischen, Formulierungen, bei denen die Zerstäubung der Formulierung durch Treibmittel unterstützt wird. Insbesondere betrifft die Erfindung Geräte, die als Inhalatoren zur Verabreichung von pulverförmigen Formulierungen verwendet werden können.

Der Einsatz von Medikamenten, die mittels Inhalatoren verabreicht werden, zielt insbesondere auf Wechselwirkungen mit speziellen Bereichen im Atmungssystem eines Patienten ab. Diese Bereiche beinhalten die Nasengänge, den Rachenraum und verschiedene Stellen innerhalb der Lunge, wie die Bronchien, Bronchiolen und Alveolen. Die Möglichkeit, Medikamente in einem dieser Zielbereiche zu verabreichen, hängt unter anderem von den aerodynamischen Durchmessern der jeweiligen Partikel oder Tröpfchen ab, die bei Anwendung des Inhalators eingeatmet werden. Derzeit geht man davon aus, dass Partikel mit aerodynamischen Durchmessern zwischen 2 und etwa 5 Mikrometern gut den Bronchien und Bronchiolen zugeführt werden können. Kleinere Partikel können potentiell in die Alveolen vordringen. Partikel, deren aerodynamische Durchmesser größer 6 Mikrometer und insbesondere größer als 10 Mikrometer sind, eignen sich typischerweise zur Ablagerung im Rachenraum und in den Nasengängen.

Bei Inhalatoren, mit denen ein Medikament in die Lunge eingebracht werden soll, ist es in der Regel wünschenswert, wenn ein hoher Anteil des verabreichten Medikaments insbesondere hinsichtlich der Teilchengröße inhalierbar ist und eine hohe Ablagerungsrate des Medikaments in der Lunge selbst erzielt werden kann. Dies hängt von mehreren Faktoren ab, wie insbesondere den Eigenschaften einer mit dem Inhalator erzeugten Sprühwolke. Diese Eigenschaften sind beispielsweise die Geschwindigkeit der Wolke, die Größen der Partikel und ihre Größenverteilung, der Anteil kleiner Partikel, die Bestandteile des Gases usw.. In der vorliegenden Erfindung weist die Sprühwolke vorzugsweise einen hohen Anteil von Partikeln mit Durchmessern von 6 Mikrometern oder kleiner, bevorzugt von kleiner 5 Mikrometern auf.
Die vorliegende Erfindung bezieht sich auf die Zerstäubung von medizinischen Formulierungen. Unter dem Begriff "medizinische Formulierung" oder "Arzneimittelformulierung" sind bei der vorliegenden Erfindung über Medikamente hinaus auch Therapeutica oder dergleichen, insbesondere also jede Art von Mitteln zur Inhalation oder sonstigen Anwendung zu verstehen. Der Begriff "Formulierung" bezieht sich hier insbesondere auf Pulver, kann aber auch Flüssigkeiten beinhalten. Dementsprechend können die Partikel sowohl fest als auch flüssig sein. Der Begriff "Flüssigkeit" umfasst neben reinen Flüssigkeiten und Lösungen zusätzlich Dispersionen, Suspensionen, Suslutionen (Mischungen aus Lösungen und Suspensionen) oder dergleichen.

Die folgende Beschreibung wird sich auf die Verabreichung von pulverförmigen Formulierungen konzentrieren, die Erfindung ist aber auch auf die Verabreichung von flüssigen Formulierungen anwendbar.

Insbesondere bezieht sich die vorliegende Erfindung auf Inhalatoren zur Einbringung von trockenem Pulver in die Lunge. Viele Pulverinhalatoren sind bereits im Handel oder anderweitig bekannt. Man unterscheidet zwischen zwei Arten von Inhalatoren: aktiven und passiven. In passiven Inhalatoren wird allein der Einatemsog des Anwenders bzw. des Patienten für die Deagglomeration des Pulvers und für den Transport des Pulvers in die Lunge verwendet. Der Transport und die Deagglomeration des Pulvers sind daher abhängig von der Flussrate, mit welcher der Patient Luft durch das Gerät einatmet. Dadurch ist der inhalierbare Anteil bzw. der Teil des Pulvers, das tatsächlich die Lunge erreicht, stark vom Einatemverhalten des Patienten abhängig und variiert von Patient zu Patient. Aktive Inhalatoren beinhalten im Gegensatz zu passiven Inhalatoren eine zusätzliche Energiequelle, welche die Deagglomeration und den Transport des Pulvers unterstützt. Das Pulver wird mit solchen Geräten aktiv zerstäubt.

Aktive Inhalatoren mit einem langjährigen hohen Marktanteil sind insbesondere konventionelle Treibmittel-getriebene Dosieraerosole (im Folgenden häufig mit der im Englischen üblichen Abkürzung MDI für "metered dose inhaler" bezeichnet). In einem MDI liegt das Medikament als Suspension oder Lösung in einem Treibmittel vor. Das Treibmittel befindet sich unter Überdruck in einem Behälter mit einem Dosierventil. Bei Aktivierung gibt das Dosierventil eine einzelne abgemessene menge (Dosis) des Medikaments in Form eines Gasstroms aus. Für MDIs geeignete Treibmittel können Hydrofluoralkane (HFA) oder andere Fluorkohlenwasserstoffe (FKW) mit niedrigen Siedepunkten beinhalten. Aufgrund der hohen Druckdifferenz zwischen dem Inneren des Treibmittel-Behälters (typischerweise um die 5 bar) und der Umgebungsluft (1 bar) tritt bei konventionellen MDIs das Treibmittel mit einer hohen Geschwindigkeit aus, die sogar in den Bereich von Überschallgeschwindigkeit kommen kann. Dadurch ist die Zeitdauer für die Entleerung der Ventilkammer und somit die Zeitdauer einer Zerstäubung so gering (typischerweise etwa 50 Millisekunden bei Abgabe von 100 Mikrolitern), dass es einem Patienten erschwert ist, sein Einatemverhalten derart an den kurzen Puls anzupassen, dass er wirklich das ganze zerstäubte Volumen einatmen kann. Außerdem tritt das Treibmittel teilweise flüssig aus dem Gerät aus. Dadurch und auch durch die große Austrittsgeschwindigkeit wird ein großer Anteil des Medikaments in einer derart groben Form abgegeben, dass er sich direkt im Mund-Rachenraum eines Patienten ablagert ohne in die Lunge transportiert werden zu können.

Die WO2004110536A1 zeigt ein Ausgabegerät für ein pulverförmiges Medikament, in der eine Aufnahme mit pulverförmigen Medikament und eine Treibmittelquelle derart in einem Gehäuse angeordnet sind, dass das Treibmittel über einen Einlass auf das pulverförmige Medikament trifft. In der Aufnahme kann sich das dabei gebildete Aerosol ausdehnen, bevor es über eine Ausgabeöffnung aus dem Gerät abgegeben wird. Für das Gerät sind Wechseleinsätze mit kombinierten Pulver- und Treibmittel-Einsätzen vorgesehen. Als mögliche Treibmittelquellen werden komprimierte Gase wie Kohlendioxid, Stickstoff oder Luft oder als konventionelle Treibmittel Fluorkohlenwasserstoffe wie HFA-134a oder HFC-227 angegeben. Im Rahmen von Versuchen für Klinikstudien werden in der Schrift WO2004110536A1 u.a. Zerstäubungsergebnisse zu Geräte-Konfigurationen mit 120 Milligramm mikronisiertem Wirkstoff gefüllten Aufnahmen und Stickstoff als Treibmittel mit Drucken von 6 bis 14 bar aufgeführt. Im Vergleich zu den geschilderten Versuchsdaten bringen handelsübliche (nicht kapselbasierte, meist passive) Pulverinhalatoren in der Regel lediglich Pulvereinheiten in der Größenordnung von 6 bis 400 Mikrogramm pro Anwendung aus (Bei der Verwendung von Kapseln sind höhere Füllmengen bekannt; so ist auch ein kapselbasierter Pulverinhalator bekannt, in dem die Kapsel 28 Milligramm aufnimmt). Die verbreiteten auf der Ausbringung mittels Treibmittel basierten MDIs bringen typischerweise nur um die 200 Mikrogramm Wirkstoffmenge pro Anwendung aus. Inhalatoren zur Ausbringung größerer Formulierungsmengen sind in diesem Zusammenhang, soweit den Erfindern bekannt, nicht im Markt erhältlich.

Die Schrift US4534345 zeigt beispielsweise einen aktiven Inhalator, der einen Treibmittel-Behälter, eine Vorratskammer mit pharmazeutisch aktiver Substanz in fester, mikronisierter Form und eine Dosis-Lade-Vorrichtung mit einer perforierten Membran beinhaltet. In einer ersten Position werden Perforationen in der Membran mit der Substanz befüllt, in einer zweiten werden die Perforationen in einen Kanal für das Treibmittel eingeschoben. Bei Betätigung einer zugehörigen Vorrichtung wird das Treibmittel aus seinem Behältnis in den Kanal abgegeben und trägt die Substanz durch eine Düse des Inhalators aus.

Die Schrift GB 1562098 A zeigt ein Inhalationsgerät mit einem Behälter, der unter Druck stehendes Treibmittel wie beispielsweise Kohlendioxid enthält, und mit einer Dosiereinheit, die Treibmittel aus dem Behälter über eine Einlassleitung erhalten kann. Die Dosiereinheit beinhaltet eine Dosiskammer, der eine Auslassleitung zugeordnet ist. Über ein Ventil wird die Dosiskammer entweder mit der Auslassleitung oder der Einlassleitung verbunden. An der Auslassleitung ist ein Gefäß mit einer Einzeldosis aktiver Substanz angeschlossen, die mitgerissen wird, wenn die in der Dosiskammer abgemessene Dosis Treibmittel durch die Auslassleitung strömt. Das Ventil und damit das Gerät werden beispielsweise über einen Atemzugs-gesteuerten Hebel aktiviert und durch eine Rückstellfeder wieder in seine Ausgangslage gebracht. Mehrere der je eine Einzeldosis aktiver Substanz enthaltender Gefäße oder Kapseln sind beispielsweise in einem Magazin ähnlich eines Patronengürtels oder eines Revolvermagazins bevorratet.

Inhalatoren für trockenes Pulver werden unterteilt in Geräte für die Verabreichung einzelner Dosiseinheiten und in Mehrdosis-Geräte.
Mehrdosis-Geräte werden des weiteren unterteilt in Geräte mit vorabgemessenen und einzeln gelagerten Dosis-Einheiten (hier wird die übliche Abkürzung "pmDPI" verwendet, welche der entsprechenden englische Bezeichnung "pre-metered Dry Powder Inhaler" entstammt) und in solche Geräte, in denen jede Pulver-Einheit innerhalb des Geräts aus einem Reservoir mittels einer Abmessvorrichtung abgemessen wird.

Die Verwendung von pmDPls hat den Vorteil, dass die einzelnen Dosis-Einheiten unter überwachten Fabrik-Bedingungen abgemessen werden und das Pulver dabei vergleichsweise einfach von Umgebungsluft und anderen äußeren Einflüssen geschützt werden kann. Bei vielen Anwendungen wird eine Formulierung in Form einer Mischung aus einem Wirkstoffs und einer Trägersubstanz wie Laktose eingesetzt. Die Laktose und/oder der Wirkstoff bzw. die Wirkstoffe neigen zur Absorption von Flüssigkeit aus der Umgebungsluft, wodurch es zur Verklumpung des Pulvers und zu Schwierigkeiten bei der DeAgglomeration bzw. der Zerstäubung und beim Transport des Pulvers in die Lunge kommt.
Die Schrift DE4106379A1 zeigt ein passives Inhalationsgerät, in dem sich das Pulver in vordosierten inhalierbaren Pulvermengen in Taschen eines biegsamen streifenförmigen Trägers befindet. Dieser Träger besteht aus einer die Taschen bildenden Trägerbahn und einer die Taschen verschließenden Abdeckbahn. Das Inhalationsgerät weist neben einer Aufnahme für den Pulverträger eine Öffnungsstation mit einer Abziehvorrichtung auf, die zum Öffnen der Taschen die Abdeckbahn und die Trägerbahn voneinander abziehen. Durch einen Pulverauslass kann Pulver aus der geöffneten Tasche angesaugt werden.

Die vorliegende Erfindung bezieht sich insbesondere auf ein so genanntes aktives Mehrdosis-Gerät zur Abgabe eines Wirkstoffs oder einer Wirkstoff beinhaltenden Formulierung zur Inhalation.
Insbesondere bezieht sich die Erfindung auf solche pmDPls, in denen Druckgas, vorzugsweise in Form von Druckluft, und/oder Treibmittel, wie vorzugsweise HFA-Gas wie Typ R134a, bei der Zerstäubung des Pulvers eingesetzt werden.
Die Schrift EP1992381A1 offenbart einen aktiven pmDPI mit einer ringförmigen schrittweise drehbaren Vorratsvorrichtung mit mehrfachen Einschüben, wobei jeder Einschub eine einzelne Dosis einer medizinischen Formulierung in einer Vorratskammer und eine Düse beinhaltet. Die Einschübe befinden sich in getrennten und versiegelten Kavitäten, die individuell für die Ausbringung der einzelnen Dosis geöffnet werden. Zur Ausbringung der Dosis wird der jeweilige Einschub über ein Verbindungselement mit einer Luftpumpe mit Faltenbalg verbunden.
Einen ähnlichen aktiven pmDPI offenbart die Schrift WO2009040044. Hierin wird zusätzlich gezeigt, dass das Verbindungselement zwischen Pumpe und Einschub eine Drossel darstellt, die zusammen mit der Luftpumpe ein Resonanzsystem darstellt. Dieses Resonanzsystem wirkt sich derart aus, dass der Druckaufbau der mittels Luftpumpe erzeugten Druckluft während der Ausbringung der Dosis nicht streng monoton steigend verläuft, sondern pulsartig moduliert ist.

Die Schrift WO2009083244A2 zeigt einen aktiven pmDPI, in dem sich die einzelnen Dosen der medizinischen Formulierung in Vorratseinheiten in einem länglichen Träger befinden. Außer einer Vorratskammer mit Formulierung umfasst jede Vorratseinheit eine Düse zur individuellen Abgabe der jeweiligen Dosis. In einer Ausführungsform werden die Düsen über das Abziehen eines Deckstreifens nach einander freigelegt. Zum Ausbringen der Formulierung aus den einzelnen Vorratseinheiten wird darüber hinaus die jeweilige zugehörige Vorratskammer mit einem Anstechelement angestochen, durch das Druckgas in die Vorratskammer geleitet wird, wodurch die Formulierung mitgerissen wird. Das Druckgas wird durch eine Luftpumpe oder alternativ aus einem Behälter mit verflüssigtem Gas bereitgestellt. In einer Ausführungsform wird die Ausbringung der jeweiligen Dosis mittels Druckluft über eine Atemzugserkennung ausgelöst.

Die Schrift GB2233236A zeigt ein MDI mit einer so genannten Atemzugstriggerung. Bei diesem Gerät wird eine abgemessene Dosis aus einem Druckbehälter mit in flüssigem Treibmittel suspendierten oder gelösten Medikament in eine Vorratskammer geleitet, in der ein Ventil in einer geschlossenen Stellung den Auslass verschließt. Das Ventil, beispielsweise ein magnetisches Tellerventil oder ein mit einer Rückstellfeder versehenes Kolbenventil, gehört zu einer über die Inhalation des Patienten gesteuerten Abgabevorrichtung.

WO 03/045483 offenbart einen Inhalator für pulverförmige, medizinische Formulierungen, der sowohl ein Dosierventil als auch ein Ventil zur Erzeugung von Pulsen aufweist.

Der vorliegenden Erfindung liegt die Aufgabe zugrunde, ein gegenüber dem Stand der Technik verbessertes Gerät zur Zerstäubung von vorzugsweise pulverförmigen medizinischen Formulierungen zur Inhalation anzugeben. Insbesondere sollte ein Gerät angegeben werden, bei dem vergleichsweise große abgemessene Substanzmengen, insbesondere größer 1 Milligramm Wirkstoff, zur Inhalation ausgebracht werden können bzw. mit dem große inhalierbare Dosismengen der medizinischen Formulierung bereitgestellt werden können. Besonders bevorzugt sollte bei der Ausgestaltung des Geräts als Inhalator der lungengängige Anteil einer als Aerosol mit diesem Gerät ausgebrachten Formulierungsmenge nur in einem geringen Maß oder gar nicht vom Einatemverhalten des Anwenders, d.h. des Patienten sein. Darüber hinaus sollte das Gerät insbesondere hinsichtlich des Aspekts der fehlerfreien Anwendung durch einen Anwender, insbesondere bzgl. der Koordination des Einatemverhaltens mit der Zerstäubung vorteilhaft gestaltet sein.

Die genannte Aufgabe wird erfindungsgemäß, gemäß der angehängten Ansprüche, gelöst durch ein Gerät und ein entsprechendes Verfahren zur Zerstäubung von medizinischen Formulierungen, wobei im Gerät die Zerstäubung durch ein Treibmittel unterstützt wird, das einer Kavität zugeführt wird, in der sich eine abgemessene Menge der Formulierung befindet, wobei das Treibmittel der Kavität in Form von mehreren aufeinander folgenden, von einander abgegrenzten Pulsen oder Schüben zugeführt wird. Das Gerät weist im Bereich der Treibmittelzuführung eine Vorrichtung auf, an derem Einlass Treibmittel anliegt und durch welche das Treibmittel durchgeleitet wird, wobei die Vorrichtung im Treibmittel ein derartiges Strömungsverhalten bewirkt, dass es aus der Vorrichtung in Form von mehreren aufeinander folgenden Pulsen oder Schüben austritt. Diese Pulse sind dabei derart von einander abgegrenzt, dass die Strömung des Treibmittels zwischen den Pulsen abreißt bzw. zum Erliegen kommt (d.h. der Druck des aus der Vorrichtung austretenden Treibmittels quasi auf Null, jedenfalls aber auf eine gegebenenfalls je nach technischer Ausstattung nicht vermeidbare minimale Restströmung absinkt). Vorteilhafte Weiterbildungen werden im Folgenden und im Detail anhand der Figuren beschrieben.

Ein Merkmal der vorliegenden Erfindung ist, dass das Treibmittel über ein Ventil zugeführt wird, das durch mehrere Öffnungs- und Schließvorgänge das anliegende Treibmittel in mehrere Pulse aufteilt. Erfindungsgemäß wird als Treibmittel verflüssigtes Treibgas verwendet. Erfindungsgemäß wird das Treibmittel dabei aus einer Kartusche, in der es verflüssigt vorliegt, über ein zur Kartusche gehörendes Dosierventil dem pulserzeugenden Ventil zugeführt. (Alternativ kann auch ein konstanter Treibgasstrom, d.h. gasförmiges Treibmittel, am pulserzeugenden Ventil anliegen.) Durch die Aufteilung des Treibmittelstoßes aus dem Dosierventil in eine Sequenz aus mehreren kurzen, zeitlich nacheinander versetzt aufeinander folgenden Treibmittelpulsen erhöht sich die Ausbringungszeit für die medizinische, vorzugsweise pulverförmige Formulierung aus dem Zerstäuber. Durch die Verlängerung der Ausbringungszeit kann ein Anwender sein Einatemverhalten leichter an die Aerosolerzeugung anpassen bzw. mit dieser koordinieren als bei der Aerosolerzeugung mittels eines einzigen Treibmittelstoßes wie beispielsweise bei einem konventionellen MDI. Die Aerosolerzeugung wird insgesamt durch die Pulsung des Treibmittels zeitlich gestreckt.
Des Weiteren hat es sich gezeigt, dass sich die Pulsung des Treibmittels mit einem Strömungsabriss des Treibmittels zwischen den Pulsen vorteilhaft auf die Ausbringung von pulverförmigen Formulierungen aus Pulverkavitäten im Zerstäuber auswirkt: Durch die Ausbringung des Pulvers mittels mehrerer Treibmittelschübe oder Treibmittelpulse werden bessere Entleerungsgrade der Pulverkavitäten erzielt. Dieser Effekt ist insbesondere bei der Ausbringung von großen Pulvermengen wie beispielsweise ab 20 Milligramm auffällig.

Vorzugsweise ist das Ventil insofern ansteuerbar, als dass Öffnungs- und Verschlusszeiten vorgegeben werden können. Gut geeignet ist beispielsweise ein ansteuerbares Magnetventil. Insbesondere werden am Ventil während des Betriebs des Geräts Öffnungszeiten im Bereich von 3 bis 30, besonders bevorzugt im Bereich von 5 bis 10 Millisekunden und Verschlusszeiten im Bereich von 50 bis 500 Millisekunden, besonders bevorzugt im Bereich von 100 bis 200 Millisekunden eingestellt. Es hat sich gezeigt, dass die Treibmittelpulse, die durch Ventilöffnungszeiten von 5 bis 10 Millisekunden mit zwischenzeitlichen Verschlusszeiten von mindestens 50, bevorzugt mindestens 100 Millisekunden erzeugt werden, in Geräten zur Zerstäubung von pulverförmigen Formulierungen eine effiziente Entleerung der die Formulierung aufnehmenden Pulverkavitäten bewirken. Dieser Effekt einer besseren Entleerung durch Pulsung des Treibmittels ist umso ausgeprägter, je größer die Pulverkavität ist. Mit gepulster Treibmittelzuführung ist so auch die Ausbringung großer Pulvermengen in nur einer Anwendung des Geräts möglich (beispielsweise wurden erfolgreich Versuche mit 50 und 75 Milligramm durchgeführt, aber auch Mengen von bis zu 100 Milligramm und mehr sind möglich). Zusammengefasst wird durch die geeignete Wahl von Länge und zeitlichem Abstand der Treibmittelpulse die Entleerung der Pulverkavität verbessert und die Zerstäubung vergleichsweise großer Pulvermengen in nur einer Anwendung des Zerstäubers ermöglicht.

Insbesondere alternativ zur Aufnahme eines ansteuerbaren Ventils in das erfindungsgemäße Gerät ist ein weiteres Merkmal der vorliegenden Erfindung, dass die Pulsung des Treibmittels durch eine Vorrichtung zur Erzeugung von Oszillationen in Fluiden, insbesondere durch einen mikrofluidischen Oszillator erzeugt wird. Die Bezeichnung "Fluid" bezieht sich hierbei sowohl auf Flüssigkeiten als auch auf Gase, insbesondere bezieht sie sich bei der vorliegenden Erfindung auf den Spezialfall, dass das Fluid ein verflüssigtes Gas ist. Bei dem mikrofluidischen Oszillator handelt es sich um eine mikrofluidische Kanalstruktur mit mindestens einer Gabelung. Je nach Auslegung des Oszillators kann die Oszillation beispielsweise entweder durch mindestens einen Steuerzulauf im Bereich der Gabelung hervorgerufen werden, so dass das Fluid abwechselnd mal in den einen oder anderen durch die Gabelung beginnenden Kanal geleitet wird, oder die Oszillation kann durch das Auftreffen zweier Strömungen aus den sich an die Gabelung anschließenden Kanälen in einem geeigneten Mischbereich bzw. in einer Oszillationskammer gebildet werden. In beiden Fällen wird der sogenannte Coanda-Effekt ausgenutzt: Ein Fluidstrahl, der aus einem Kanal in einen Erweiterungsbereich austritt, schmiegt sich an diejenige Wand an, die in Bezug auf die Achse des Strahls weniger geneigt ist. Dies ist beim Coanda-Effekt die stabile Orientierung des Strahls. Wenn jedoch die Geometrie des entsprechenden Geräts bzw. der entsprechenden Kanalstruktur (beispielsweise bezogen auf eine an einen Erweiterungsbereich anschließende Gabelung) symmetrisch ist, kann sich der Strahl an die eine oder andere Wand anschmiegen und bleibt in dieser Orientierung bis andere Faktoren wie z.B. Druckvariationen, Wirbel oder Querströmungen einen Orientierungswechsel zum Anschmiegen an die andere Wand hin veranlassen.
Insbesondere bei der Verwendung von Flüssiggas als Treibmittel kann hierbei zusätzlich der Effekt ausgenutzt werden, das die Flüssigkeit beim Durchströmen der Kanalstruktur aufgrund ihres geringen Siedepunktes bereits teilweise in die Gasphase übergeht. Durch die entstehenden Siedeverzüge bilden sich somit immer wieder Gasblasen, insbesondere an Stellen wie beispielsweise in Oszillationskammern oder Mischbereichen, wo sich bei Verwirbelungen DichteUnterschiede im Fluid und somit Stellen mit unterschiedlichem Verdampfungsverhalten ergeben. Solche Gasblasen führen dann ihrerseits zu einer puls- oder schubartigen Ausbringung des Treibmittels aus einem Auslass eines solchen Mischbereichs.
Die Verwendung eines solchen mikrofluidischen Oszillators in einem Treibmittelbetriebenen Gerät zur Zerstäubung hat den Vorteil, dass aufgrund der kleinen Baugröße eines mikrofluidischen Oszillators die Baugröße des Geräts zum Zerstäuben der medizinischen Formulierung nur vergleichsweise wenig vergrößert wird. Auf diese Weise kann eine Vorrichtung zum Pulsen des Treibmittels in Handgeräten eingebaut werden, die bei ihrer Verwendung ohne externe Vorrichtungen zur Ansteuerung auskommen.

Ein weiteres Merkmal der vorliegenden Erfindung ist, dass das Treibmittel zur Ausbringung der medizinischen, vorzugsweise pulverförmigen Formulierung aus einer Kavität vor der Zuführung zu dieser Kavität durch einen Verdampfer oder Wärmetauscher geleitet wird. Der vorzugsweise aus Metall gefertigte Verdampfer weist einen Hohlraum mit einem Einlass und einen Auslass für das Treibmittel auf. Der Einlass des Verdampfers ist vorzugsweise einstückig mit einem Bauteil ausgebildet, das den Anschluss eines für die Pulserzeugung benutzten Ventils oder Oszillators bildet.
Der Verdampfer bewirkt, dass das Treibmittel, das z.B. in einer Vorratskartusche flüssig vorliegt, vollständig oder nahezu vollständig in den gasförmigen Zustand überführt wird, bevor es der Kavität mit der Formulierung zugeführt wird. Das in einer konventionellen Kartusche unter Druck flüssig gehaltene Treibmittel verdampft unter Normaldruck in der Regel bei negativen Temperaturen auf der Celsius-Temperatur-Skala. Wird es in den Hohlraum des Verdampfers eingeleitet, kann es dort expandieren und wechselt vom flüssigen in den gasförmigen Zustand. Insbesondere bei der Verwendung pulverförmiger Formulierungen wird dadurch verhindert, dass flüssiges Treibmittel das Pulver verklumpt und die Zerstäubung des Pulvers dadurch beeinträchtigt wird. Unter Verwendung des Verdampfers erhöht sich somit der lungengängige Anteil der mittels Treibmittelausbringung erzeugten Aerosolpartikel.

Ein weiteres Merkmal der vorliegenden Erfindung ist, dass der Verdampfer in seinem Innern Wärmetausch-Elemente beinhaltet. Die Wärmetausch-Elemente unterstützen bzw. beschleunigen die Verdampfung des flüssigen Treibmittels im Verdampfer, in dem sie Wärme an das an ihnen entlang strömende Treibmittel abgeben. Diese Wärmetausch-Elemente sind vorzugsweise aus Metall und weisen eine relativ große Oberfläche auf, was ebenfalls den Verdampfungseffekt begünstigt. Vorzugsweise haben die Wärmetausch-Elemente hierzu die Form von Kugeln und/oder Drähten.

Ein weiteres Merkmal der vorliegenden Erfindung ist, dass im Innern des Verdampfers die Bauteile des Verdampfers derart gestaltet sind, das sie für das durch den Verdampfer strömende Treibmittel einen möglichst geringen Strömungswiderstand darstellen. Dadurch kann nahezu die volle Geschwindigkeit der Treibgasströmung für die Pulverzerstäubung genutzt werden. Zur Reduzierung des Strömungswiderstandes tragen bei:
- die rotationssymmetrische Gestalt des Verdampfers,
- konusförmige Übergänge im Einlass- und Auslassbereich des Hohlraums des Verdampfers,
- Kugelstruktur der Wärmetausch-Elemente im Verdampfer und/oder
- eine derartige Bemessung der Wärmetausch-Elemente, dass es durch sie zu keiner Verlegung von Einlass oder Auslass des Verdampfers kommt und dass ihre Zwischenräume gut durchströmbar sind.

Ein weiteres Merkmal der vorliegenden Erfindung ist, dass die Zuleitung von Treibmittel in die Kavität mit der Formulierung und die Achse eines vorzugsweise geradlinigen Düsenkanals an der gleichen Stelle, bevorzugt mittig, und unter dem gleichen Winkel relativ zum Boden der Kavität eintreffen. Bei dieser Anordnung erstreckt sich die Achse des Düsenkanals, durch den die Formulierung mittels Treibmittel aus der Düse des Geräts bzw. des Zerstäubers ausgebracht wird, gerade in der Richtung, in welche der Treibmittelstrom von der Pulverkavität aus zum größten Teil reflektiert wird. Auf diese Weise stößt der mit Formulierung behaftete Treibmittelstrom auf seinem Weg nach draußen nicht unnötig an die Wände der Kavität. Die Kavität wird besser entleert bzw. es kommt nicht oder kaum zu Ablagerungen von Formulierung neben dem Einlass des Düsenkanals. Ein Winkel von 45° ist hierbei für den Aufbau eines kompakten Zerstäubers vorteilhaft, beispielsweise wenn mehrere Kavitäten entlang eines kreisförmigen Radius nacheinander in die Position zur Ausbringung der Formulierung gebracht werden. Aber auch etwas geringere Winkel wie z.B. 30° können insbesondere bei länglichen Kavitäten vorteilhaft sein, da dann mehr Formulierung vom Treibmittel sozusagen durchstoßen werden muss, bevor der Treibmittelstrom reflektiert wird. Dieser Reflektionswinkel sollte an die optimale Gestaltung von Kavität und gegebenenfalls an einen Austauschmechanismus für mit Formulierung gefüllte Kavitäten angepasst werden.

Ein weiteres Merkmal der Erfindung ist, dass die Pulverkavität stromlinienförmig gestaltet ist. Vorzugsweise weist sie eine Tropfenform auf (ähnlich einem längs aufgeschnittenen Tropfen) und/ oder weist einen Napf mit einer tropfenförmigen Öffnung auf. Hierbei ist die Pulverkavität bevorzugt so in dem Zerstäuber angeordnet, dass die Treibmittelzuführung oder ein Lufteinlass in der Nähe des breiten Bauchs der Tropfenform oder in Strömungsrichtung gesehen am unteren Rand des Bauchs befindet und der engere Teil der Tropfenform auf den Düsenkanal zuläuft oder das enger werdende Ende der Tropfenform direkt in den Düsenkanal mündet. Besonders bevorzugt weist der Boden des Napfs eine Schräge auf, welche die in die Pulverkavität eingeleitete Strömung direkt in Richtung Düsenkanal leitet. Eine derartige Gestaltung der Pulverkavität führt zu einer quasi vollständigen Entleerung bei Anwendung. Dadurch kann der Zerstäuber außerdem bezogen auf die verwendete medizinische Formulierung im Vergleich zur Verwendung anderer Pulverkavitäten mit niedrigeren Füllvolumina verwendet werden. Ein weiteres Merkmal der vorliegenden Erfindung ist, dass das Gerät im Falle einer Ausführungsform als Inhalator zur Ausbringung mehrerer einzeln abgemessenen Mengen der Formulierung dient. Hierzu befinden sich die einzelnen Mengen der Formulierung, z.B. einzelne Pulvereinheiten, bevorzugt in den Kavitäten eines Blisterbands. Die Kavitäten werden dann nacheinander z.B. durch eine Weiterbewegung des Blisterbands in eine Entnahmeposition im Treibgasstrom gebracht. Zuvor sind sie z.B. über einen Mechanismus, durch den die jeweilige Kavität beispielsweise angestochen wird oder durch den vorzugsweise eine die Kavität verschließenden Deckfolie abgezogen wird, geöffnet worden. Die Bevorratung der Kavitäten entlang eines Blisterbands hat den Vorteil, dass durch Rollen bzw. Wickeln des Bandes viele Kavitäten auf kleinem Raum bevorratet werden können. Besonders bevorzugt wird der Transport des Blisterbandes im Inneren des Geräts durch eine Bewegung außen am Gerät gesteuert, wie insbesondere das Öffnen oder Schließen einer Mundstückabdeckung.
Ein weiteres Merkmal der vorliegenden Erfindung ist, dass die Ausbringung der Formulierung aus der Kavität durch eine Düse erfolgt, deren Auslassöffnung in ein in Strömungsrichtung verlängertes Mundstück einmündet. Bevorzugt ist dabei der Überstand des Mundstücks im Vergleich zum Düsenauslass länger als der Düsenkanal in der Düse. Bevorzugt ragt das Mundstück 40 bis 120 Millimeter, besonders bevorzugt 40 bis 70 Millimeter über das Ende der Düse hinaus. Bei einem derart bemessenen Überstandsbereich ist einerseits der Anteil des lungengängigen Feinanteils des ausgebrachten Aerosols gegenüber Geräten mit kleineren Überständen erhöht und andererseits ist der Überstand noch nicht so groß, als das es zu einer übermäßigen Bildung von Ablagerungen innen im Mundstück kommt. Bei den größeren Überstandsbereichen, insbesondere bei Überständen von 70 bis 120 Millimeter, nimmt der Anteil der inhalierbaren Dosis weiter zu, es muss lediglich gegebenenfalls eine regelmäßige Reinigung des Mundstücks vorgesehen werden.
Zur Ausbildung einer für die Inhalation des gebildeten Aerosols günstigen Aerodynamik im Mundrohr weist das Mundstück des Weiteren geräteseitig (d.h. auf der Seite, die der Stelle entgegengesetzt liegt, an der im Falle eines Geräts zur Inhalation an Anwender seine Lippen ansetzen würde) mindestens eine, bevorzugt 1 bis 4 Einlassöffnung auf. Bevorzugt ist die Einlassöffnung am Mundstück so gestaltet, dass es zu einer Bypass-Luftströmung nahe dem Auslass der Düse kommt, die besonders bevorzugt den aus der Düse austretenden mit Formulierung beladenen Treibmittelstrom umhüllt. Es zeigt sich, dass die Aerodynamik weiterhin günstig beeinflusst wird, wenn der innere Durchmesser des Mundstücks an der Stelle des Düsenauslasses deutlich größer, insbesondere fünfmal, so groß wie der Duchmesser der Öffnung des Düsenauslasses ist.

Ein weiteres Merkmal der vorliegenden Erfindung ist, dass das Gerät im Falle einer Ausführungsform als Inhalator eine Atemzugstriggerung aufweist, welche die Zuführung von Treibmittel in den Verdampfer auslöst. Bevorzugt befindet sich ein Schaltelement der Atemzugstriggerung z.B. in Form eines Strömungssensors im Bereich der Einlassöffnungen des Mundstücks und/oder in einem an die Einlassöffnungen angeschlossenen Kanal.

Die einzelnen Merkmale der vorliegenden Erfindung können unabhängig voneinander genutzt oder miteinander kombiniert werden.
Weitere Vorteile, Merkmale, Eigenschaften und Aspekte der vorliegenden Erfindung ergeben sich aus den Ansprüchen und der folgenden Beschreibung bevorzugter Ausführungsformen anhand der Zeichnungen. Die Zeichnungen zeigen in:
Fig. 1 einen schematischen Schnitt durch die Zerstäubungseinheit eines Zerstäubers mit Treibmittelzuführung,
Fig. 2 einen als Testvorrichtung gestalteten Zerstäuber mit Treibmittelzuführung,
Fig. 3a einen schematischen Schnitt durch einen Teil der Treibmittelzuführungsvorrichtung zu Fig. 2 und Fig. 3b einen schematischen Schnitt durch zwei Einzelbauteile aus der Treibmittelzuführung,
Fig. 4a einen als Testvorrichtung gestalteten, erfindungsgemäßen Zerstäuber mit gesteuertem Ventil in der Treibmittelzuführung, Fig. 4b ein Flussbild bezogen auf die Abläufe im Zerstäuber aus Fig. 4a, und Fig. 4c ein Flussbild bezogen auf die Anbindungen eines alternativen Treibgas-betriebenen Zerstäubers und Fig. 4d zeigt ein Diagramm mit extrapolierten Messergebnissen zur Pulverkavitätentleerung in Abhängigkeit von am Ventil eingestellten Öffnungs- und Verschlusszeiten t₁ und t₂ für eine Anordnung entsprechend Fig. 4a,
Fig. 5a, b, c und d verschiedene mikrostrukturierte Kanalstrukturen zur Pulserzeugung in Strömungen,
Fig. 6a einen schematischen Schnitt durch die Zerstäubungseinheit eines Geräts, wobei in Fig. 6b und ebenfalls in Schnittansicht in Fig. 6c eine in diesem Gerät eingesetzte Düse dargestellt ist,
Fig. 7 verschiedene Pulverkavitäten zur Verwendung mit einem Zerstäuber gemäß Fig. 1 und Fig. 2: Fig. 7a einen Träger mit einer wannenförmigen Pulverkavität, Fig. 7b einen Träger mit tropfenförmiger Pulverkavität in Aufsicht und Fig. 7c die tropfenförmige Pulverkavität in schematischer Schnittansicht, und
Fig. 8 einen erfindungsgemäßen Inhalator als Handgerät, wobei Fig. 8a den Inhalator von außen mit geschlossener Mundstückabdeckung, Fig. 8b in schematischer Schnittansicht und Fig. 8c von außen mit geöffnetem Mundstück zeigt.

In den Figuren werden für gleiche oder ähnliche Teile dieselben Bezugszeichen verwendet, wobei entsprechende oder vergleichbare Eigenschaften und Vorteile erreicht werden, auch wenn eine wiederholte Beschreibung weggelassen ist.

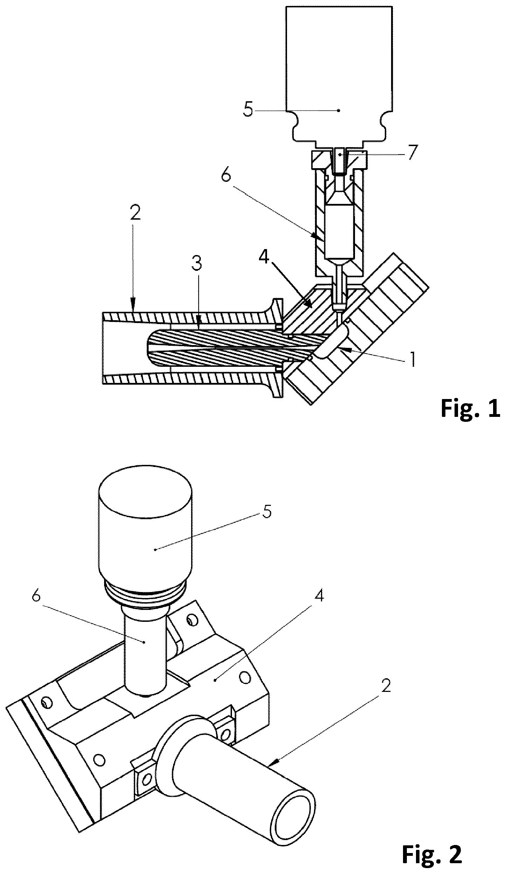

Fig. 1 zeigt in schematischer Schnittdarstellung die Funktionsweise eines Geräts, das insbesondere für die Zerstäubung von Pulvern geeignet ist. Der Aufbau des dargestellten Geräts bzw. Zerstäubers eignet sich sowohl für einen Inhalator als auch vor allem für eine Testvorrichtung zur Überprüfung der Funktionsweise einzelner jeweils austauschbar gehaltenen Komponenten des Inhalators. Ein solcher als Testvorrichtung gestalteter Zerstäuber ist in Fig. 2 abgebildet.

Bei der Zerstäubung von Pulvern wird hierbei Treibmittel aus einer Kartusche (5) direkt oder vorzugsweise nach vollständiger Durchführung durch einen Verdampfer (6) oder Wärmetauscher in eine Pulverkavität (1) geleitet, auf die eine Düse (3) aufgesetzt ist, die wiederum in ein Mundstück (2) einmündet. Bei der hier bevorzugten Verwendung eines Verdampfers (6), sollte dieser so im System eingebaut sein, dass ausgeschlossen ist, dass ein Teil des aus dem Ventil an der Kartusche (5) abgegebenen Treibmittels über einen Bypass am Verdampfer (6) vorbei in die Pulverkavität (1) gelangen kann. Das Treibmittel treibt das Pulver aus der Pulverkavität (1) durch die Düse (3) aus. Saugt gleichzeitig ein Anwender bzw. Patient am Mundstück (2) Luft an, so wird das Pulver beim Austritt aus der Düse (2) von der Einatemluft mitgerissen, die an der Düse vorbei durch das Mundstück (2) in die Lunge strömt. Das Pulver gelangt so durch den Atemsog des Patienten in dessen Lunge.
Sowohl die Testvorrichtung als auch ein gemäß gleicher Funktionsweise aufgebauter Inhalator wird vorzugsweise so bedient, dass der Boden der Kartusche (1) nach oben zeigt, d.h. dass der Ventilstamm (7) der Kartusche (1) nach unten gerichtet ist. Eine solche Bedienung ist analog zur Bedienung eines konventionellen Dosieraerosols (im Folgenden wird häufig mit der aus dem Englischen üblichen Abkürzung MDI bezeichnet). Das Treibmittel wird mittels eines zur Kartusche gehörenden Ventils (ebenfalls in Analogie zum konventionellen MDI) dosiert, wobei die Abgabe einer Einheit des Treibmittels beispielsweise über Druck auf den Boden der Kartusche (5) ausgelöst wird.

Bei der in Fig. 2 gezeigten Testvorrichtung besteht der Zerstäuber aus einzelnen Modulen. Die funktionsrelevanten Module sind hierbei insbesondere die Kartusche (5), der Verdampfer (6), die Düse (3), das Mundstück (2) und eines, das die Pulverkavität (1) bildet. Zusätzlich kann im Fall der Testvorrichtung noch ein Anschlusstück (4) hinzukommen, an dem andere Module insbesondere die Düse (3), die Pulverkavität (1) und der Verdampfer (6) oder die Kartusche (5) austauschbar befestigt und in fluidische Beziehung zueinander gebracht werden können.
Die Testvorrichtung bietet den Vorteil, dass die in die Testvorrichtung eingesetzten Module variiert werden können und die Auswirkung der jeweiligen Variation auf das Zerstäubungsverhalten bestimmt werden kann. Bevorzugt wird außerdem ein System zur Strömungserzeugung, z.B. eine laborübliche Steuerwand mit konventioneller Pumpe, an die Testvorrichtung angeschlossen, so dass verschiedene Saugstärken am Mundstück (2) nachgestellt werden können. Die Zerstäubung des jeweils in der Testvorrichtung eingesetzten Pulvers kann mit den gleichen Methoden wie die Zerstäubung aus Inhalatoren untersucht werden, insbesondere mittels Laserbeugungsmethoden, Hochgeschwindigkeitskameras und Kaskadenimpaktoren. Die in dieser Schrift zugrunde liegenden Messergebnisse wurden mit einem gemäß den Vorgaben des Europäischen Arzneibuchs (Version 7.0) gestalteten Kaskadenimpaktor ("Apparatus E" gemäß Kapitel 2.9.18 im Europäischen Arzneibuch) zur Bestimmung des Feinanteils der ausgebrachten Dosis ermittelt (wobei zusätzlich bei verschiedenen Flussraten gemessen wurde). Dieser Feinanteil wird bezogen auf Partikelgrößen mit Durchmessern kleiner als 5 Mikrometern und wird im Folgenden zuweilen auch mit der Abkürzung FPD bezeichnet (hergeleitet aus der oft angewandten englischen Bezeichnung "Fine Particle Dose"). Bei den Messungen mit dem hierzu eingesetzten Kaskadenimpaktor wurden die Wirkstoff-Ablagerungen auf den einzelnen, die Kaskadenstufen bildenden Schalen und der Anschluss des Kaskadenimpaktors jeweils pro Messung individuell gewaschen und die sich ergebenden Lösungen wurden mittels Hochdruck-Flüssigkeits-Chromatographie untersucht. In den einzelnen Kaskadenstufen werden während der Messung unterschiedliche Größenklassen der zerstäubten Partikel gesammelt, so dass mittels der Chromatographie der Lösung aus der jeweiligen Kaskadenstufe die jeweiligen Anteile der Dosis pro Größenklasse bestimmt werden können.
Die Testvorrichtung wird bevorzugt für die Überprüfung der Zerstäubung von pulverförmigen medizinischen Formulierungen eingesetzt. Bevorzugte Formulierungen bestehen aus strahlgemahlenem und gesiebtem Wirkstoff, der mit Laktose gemischt wird. Beliebige Mischungsverhältnisse bis hin zu reinem Wirkstoff können mit der Testvorrichtung überprüft werden.
Für die meisten Messungen wurde in der Testvorrichtung eine Pulverkavität (1) mit einem Fassungsvermögen von etwa 0,2 Millilitern verwendet, in die meist 50 Milligramm einer auf Laktose basierten Formulierung mit 32,5% einer aktiven pharmazeutischen Substanz (ein in der Entwicklung befindlicher Wirkstoff) eingefüllt wurden. Für das hier beschriebenen Zerstäubungskonzept können jedoch auch Pulverkavitäten (1) mit Pulvermengen von bis zu 100 Milligramm befüllt werden; getestet wurden beispielsweise 21 Milligramm, 60 Milligramm, 70 Milligramm und 75 Milligramm (einige davon mit einer Formulierung, die 98% Fenoterol-HBr beinhaltet, da sich dieser Wirkstoff gut für Extremalprüfungen der Zerstäubung von Inhalatoren eignet). Die Größe der Pulverkavität (1) kann an die Menge des aufzunehmenden Pulvers angepasst werden. Auch Pulvermengen bis hin zu 250 Milligramm sind möglich bei entsprechenden Anpassungen der Pulverkavität (1) und der Treibmittelzufuhr (hin zu größeren Drücken und umfangreicheren Treibmittelstößen) möglich. Die Form der Pulverkavität ist vorzugsweise an das angeschlossene Düsenkonzept angepasst.
Die Ausbringung solch im Vergleich zu handelsüblichen Pulverinhalatoren hoher Pulvermengen wird in diesem Fall durch die Verwendung von Treibmitteln ermöglicht. Bevorzugt werden hierbei Hydrofluoralkane (so genannte HFAs) verwendet. Besonders bevorzugt wird hier als Treibmittel HFA R134a (Norfluran oder 1,1,1-Trifluorethan bzw. 1,1,1,2-Tetrafluorethan) in der Kartusche (1) eingesetzt, wobei hier bevorzugt Kartuschen mit Dosierventilen verwendet werden, die 100 Mikroliter pro Aktivierung abgeben. Je nach Größe der eingesetzten Formulierungsmenge kann die Größe des Ventils angepasst werden, wobei, handelsübliche Größen wie beispielsweise 25, 50, 100, 180 bis hin zu 500 Mikrolitern verwendet werden können. So wurden im Rahmen der hier vorgestellten Ergebnisse bei kleineren Formulierungsmengen ebenfalls kleinere Treibmitteleinheiten verwendet, wie beispielsweise 50 Mikroliter Treibmittel bei 21 Milligramm Pulvermenge. Bevorzugt wurden die handelsüblichen Ventile mit metallischen Behältern verbunden, insbesondere wurden hier bei der Kartuschenherstellung die Ventile mit 10 Milliliter fassenden Aluminiumcontainern vercrimpt. Die Kartuschen wurden mit dem Treibgas R134a gefüllt. In der Kartusche (5) liegt das Treibmittel - wie auch in handelüblichen Dosieraerosolen (MDIs) - in verflüssigter Form vor. Es verdampft unter Normaldruck bei -26,3° C und entwickelt bei 20° C einen Dampfdruck von 5,7 bar. Aber auch andere Treibmittel wie andere Hydrofluoralkane wie beispielsweise HFA 227ea (Apafluran oder 1,1,1,2,3,3,3-Heptafluoropropan), flüssiger Stickstoff oder konventionelle Treibmittel wie Halogenkohlenwasserstoffe können bei diesem Prinzip eingesetzt werden.

Die in Fig. 2 gezeigte Testvorrichtung wird bei einer Ausrichtung der Kartusche (5) senkrecht nach oben, mit nach unten gerichteten Ventilstamm (7) verwendet. Wenn beispielsweise durch Drücken auf den nach oben gerichteten Boden der Kartusche Treibmittel freigesetzt wird, fließt das Treibmittel zunächst in den Verdampfer (6) und von da aus als Treibgas über eine Zuleitung im Anschlussstück (4) in die Pulverkavität (1). Das Treibgas treibt dann das Pulver der Pulverkavität (1) durch die Düse (3) aus. Durch die Verwendung des Treibmittels HFA R 134A mit einem Druck von etwa 6 bar bei Raumtemperatur werden vorteilhafterweise im System größere Flussgeschwindigkeiten als mit konventionellen Fluorkohlenwasserstoffen erzielt, die abgefüllt lediglich Drücke im Bereich von 2 bis 4 bar aufweisen.
Der Verdampfer (6) bewirkt, dass das mit dem Dosierventil abgegebene, abgemessene Treibmittel, das in der Kartusche (5) flüssig vorlag, komplett in den gasförmigen Zustand überführt wird, bevor es der Pulverkavität (1) zugeführt wird.

Das Treibmittel wird dabei gewissermaßen getrocknet. Dadurch wird verhindert, dass flüssiges Treibmittel das Pulver verklumpt. Verklumptes Pulver lässt sich wesentlich schlechter dispergieren und die Lungengängigkeit der bei der Zerstäubung erzeugten Aerosol-Partikel verschlechtert sich. Videoaufnahmen mit einer Hochgeschwindigkeitskamera, mit der durch einen transparenten Boden der Pulverkavität (1) das Innere der Pulverkavität (1) während der Zuleitung von Treibmittel beobachtet wurde, belegen, dass es ohne die Verwendung eines Verdampfers (6) zu besagter Verklumpung kommt, während bei Verwendung des im Folgenden im Detail beschriebenen Verdampfers keine Agglomerate oder Verklumpungen mehr zu erkennen waren.

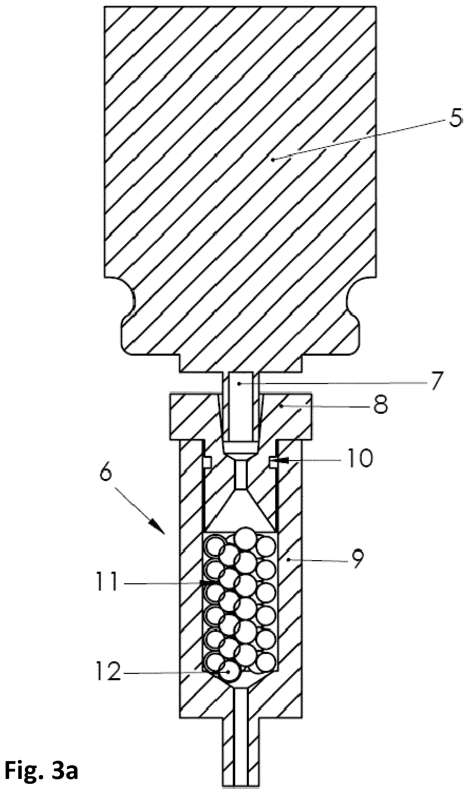

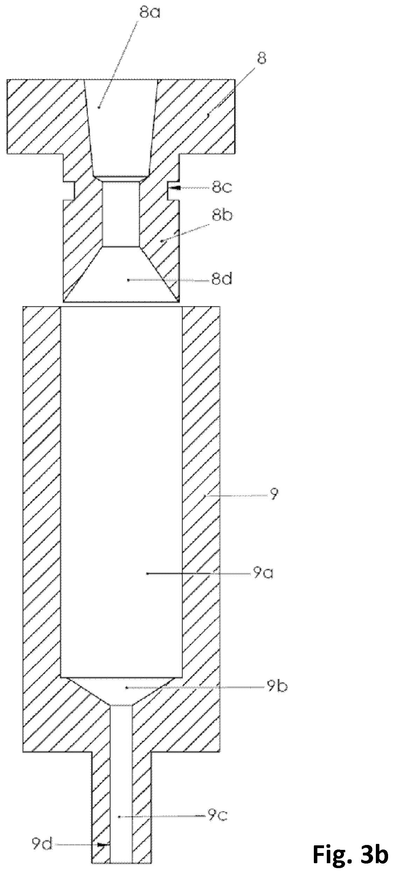

Fig. 3 zeigt einen möglichen Aufbau eines solchen Verdampfers (6). In der gezeigten Ausführungsform für den Einsatz in einer Testvorrichtung ist der Einlass des Verdampfers (6) so gestaltet, dass er einerseits eine Aufnahme (8a) für einen handelsüblichen Ventilstamm (7) einer Kartusche (5) aufweist und andererseits der Auslass (9c) so gestaltet ist, dass das Äußere des Verdampfers (6) an dieser Stelle gerade die Form eines Stammes (9d) mit den gleichen Abmessungen wie bei einem handelsüblichen Ventilstamm aufweist, beispielsweise einen Durchmesser von 2 Millimetern. Dementsprechend weist auch das Anschlussstück (4) eine Ventilstammaufnahme auf, an der für Versuchszwecke wie hier gezeigt der Verdampfer (6) oder wahlweise direkt die Kartusche (5) angeschlossen werden kann.
Der dargestellte, vorzugsweise rotationssymmetrisch gestaltete Verdampfer (6) beinhaltet einen Körper (9) mit einem vorzugsweise zylinderförmigen Hohlraum (9a). Der Boden des Hohlraums ist vorzugsweise als Trichter (9b) gestaltet, von dessen Mitte der Auslass (9c) als Kanal abgeht. Verschlossen wird der Hohlraum (9a) des Verdampfers durch einen Deckel (8), dessen Flansch (8b) in den oberen Bereich des Hohlraums (9a) eingetaucht wird. Vorzugsweise werden der Körper (9) und der Deckel (8) des Verdampfers (6) mittels einer Dichtung (10) gegeneinander abgedichtet. Diese Dichtung (10) ist beispielsweise bei einem rotationssymmetrischen Verdampfer (6) ein ringförmiges Bauteil aus einem elastomeren Material. Die Dichtung wird beispielsweise in eine Sicke (8c) am Flansch (8b) des Deckels (8) eingelegt. Der Deckel weist eine Ventilstammaufnahme (8a) passend zur Aufnahme eines Ventilstamms einer handelsüblichen Kartusche (5) und eine Durchführung auf, die sich bevorzugt über einen Innenkonus (8d) zum Hohlraum (9a) hin öffnet. Bevorzugt wird am den oberen Bereich des Ventilstamms (7) eine zusätzliche Dichtung, beispielsweise in Form eines O-Rings, angebracht, so dass keine Umgebungsluft entlang des Ventilsstamms (7) über die Ventilstammaufnahme (8a) ins System gelangt.
Die Wirkung des Verdampfers (6) basiert auf Wärmetausch-Mechanismen. Der Verdampfer (6) selbst besteht vorzugsweise aus Metall und der Hohlraum ist bevorzugt ebenfalls mit vorzugsweise metallenen Bauteilen gefüllt, die im Folgenden als Wärmetausch-Elemente bezeichnet werden. Das in der Kartusche (5) unter Druck flüssig gehaltene Treibmittel verdampft unter Normaldruck, im Ausführungsbeispiel bereits ab etwa -25°Celsius. Kann es also im Hohlraum des Verdampfers (6) expandieren, so wechselt es vom flüssigen in den gasförmigen Zustand. Die Wärmetausch-Elemente unterstützen bzw. beschleunigen diese Verdampfung, in dem sie Wärme an das an ihnen entlang strömende Treibmittel abgeben. Die Wärmetausch-Elemente haben hierzu bevorzugt eine möglichst große Oberfläche. Hierdurch wird die gesamte innere Oberfläche des Verdampfers, d.h. die Kontaktfläche für den Wärmetausch-Mechanismus, erhöht und das Totvolumen, d.h. der freie Raum innerhalb des Verdampfers, verkleinert. Als Material werden für alle Bauteile des Verdampfers Metalle bevorzugt, da sie eine hohe Wärmeleitfähigkeit aufweisen und dadurch die Verdampfung des auf sie auftreffenden Treibmittels begünstigen. Möglich ist die Verwendung der meisten bei Raumtemperatur fester Metalle, wobei Edelstähle besonders bevorzugt sind, da sie zu den meisten Treibmitteln insofern eine gute Kompatibilität aufweisen, als dass keine Bestandteile des Metalls vom Treibmittel aufgenommen und der Zerstäubung zugeführt werden. Edelmetalle wie Silber und Gold wären ebenfalls gut geeignet, scheiden aber aus Kostengründen meistens aus. Sowohl für den Körper (9) des Verdampfers (6) als auch für Wärmetausch-Elemente in seinem Hohlraum (9a) werden hier konkret Aluminium, Edelstahl oder Kupfer als Materialien bevorzugt. In einer bei Versuchen verwendeten Ausführungsform ist der Körper (9) des Verdampfers (6) aus Aluminium, und der Hohlraum (9a) ist mit Kugeln (11) aus Edelstahl gefüllt. Je nach Verdampfungsverhalten des gewählten Treibmittels kann es gegebenenfalls ausreichen, wenn lediglich die Wärmetausch-Elemente aus Metall sind und der Körper (9) selbst aus Kunststoff ist. Dies bietet Kostenvorteile bei der Produktion. Der Verdampfer kann hinsichtlich seiner Länge und damit auch der Anzahl an Wärmetausch-Elementen variiert werden, um an die verwendete Menge Treibmittel angepasst zu werden. So kann bei der Verwendung von kleinen Treibmittelmengen ein kleinerer bzw. kürzerer Verdampfern verwendet werden. Im Rahmen von Untersuchungen wurden beispielsweise in Verbindung mit Dosierventilen, die 50 Mikroliter Treibmittel abgeben, ein Verdampfer (6) mit 6 Millimeter langem Innenraum und gefüllt mit 27 Stahlkugeln von 2 Millimeter Durchmesser eingesetzt. In Verbindung mit 100 Mikroliter-Ventilen wurde ein 12 Millimeter langer Verdampfer (6) mit 48 Kugeln (11) eingesetzt. Dieses Verhältnis von Verdampferlänge bzw. Anzahl von Kugeln (11) zum Volumen des eingesetzten Dosierventils ist beliebig skalierbar - auch für größere Dosierventile.
Damit keine der Kugeln (11) den Auslass (9c) im Boden des Trichters (9b) verlegt, liegt beim konkreten Ausführungsbeispiel im Trichter (9b) ein gewundener Draht (12) beispielsweise aus Kupfer, der die Kugeln (11) vom Auslass (9c) fernhält. Wahlweise können die Metallkugeln auch ganz durch einen langen, dünnen gewickelten Metalldraht im Hohlraum (9a) ausgetauscht werden. Im Ausführungsbeispiel haben die Kugeln (11) einen Durchmesser von 2 Millimetern, und dem steht ein Auslass (9c) mit einem Durchmesser von 1 Millimeter gegenüber.
Insgesamt ist die Gestalt bzw. die Anordnung der Bauteile so gewählt, dass der Verdampfer (6) einerseits zwar eine möglichst große innere Oberfläche für eine effiziente Verdampfung des Treibmittels aufweist, andererseits die Füllung mit Wärmetausch-Elementen hinreichend viele kleine freien Querschnitte aufweist, so dass sein Strömungswiderstand nicht so hoch wird, d.h. dass er das durch ihn durch geleitete Treibmittel nicht zu sehr verlangsamt. In diesem Zusammenhang wird hier bezogen auf Luft ein Strömungswiderstand von um die 465000 √N*s/m⁴ ± 10% bevorzugt (für Treibmittel sind noch geringere Strömungswiderstände als Luft zu erwarten). Dies entspricht einem zustande kommenden Fluss von 10 Liter pro Minute bei einem Druckabfall von 6 Kilopascal (ein geringerer Fluss als 5 Liter pro Minute bei einem Druckabfall von 6 Kilopascal würde beispielsweise einen ungünstigeren Strömungswiderstand darstellen, das Treibgas würde auf seinem Weg durch den Verdampfer dann deutlich abgebremst). Der Strömungswiderstand des Verdampfers wird durch seine Geometrie und durch die Größe und Form der in ihm enthaltenen Wämetausch-Elemente beeinflusst. Bei der hier bevorzugten Auslegung ermöglichen die Zwischenräume zwischen den Kugeln eine gute Durchströmbarkeit des Verdampfers, die Konturen in Übergangsbereichen sind durch die Verwendung von Konus-Strukturen strömungstechnisch günstig und die Kugelform der Wärmetausch-Elemente trägt ebenfalls dazu bei, dass sich keine oder nur geringe Turbulenzen in der Strömung bilden. Dadurch kommt es im Verdampfer nur zu einer geringen Druckabsenkung des Treibmittels, der Gasstrom wird also nur wenig verlangsamt. Dies ist in sofern von Vorteil, als dass eine hohe Geschwindigkeit des Treibmittels wesentlich für eine gute Dispersion des Pulvers ist.
Die Geschwindigkeit des Treibmittels bei Eintritt in die Pulverkavität (1) kann durch die Größe der Durchmesser der Zuführungskanäle beeinflusst, insbesondere gedrosselt werden. Im Fall der Testvorrichtung können verschiedene Anschlussstücke (4) mit unterschiedlich breiten Zuführkanälen getestet werden. Bei den hier zugrunde liegenden Messergebnissen wurden Kanaldurchmesser im Bereich von 0,2 bis 2 Millimeter zwischen Verdampfer (6) und Pulverkavität (1) getestet, wobei sich Durchmesser im Bereich von 1 bis 2 Millimeter als besonders geeignet erwiesen. Im Falle eines für Massenproduktion geeigneten Inhalators ist aus Kostengründen anzuraten, den Verdampfer (6) mit geeignet bemessenem Auslass (9c) direkt an die Pulverkavität (1) anzuschließen.

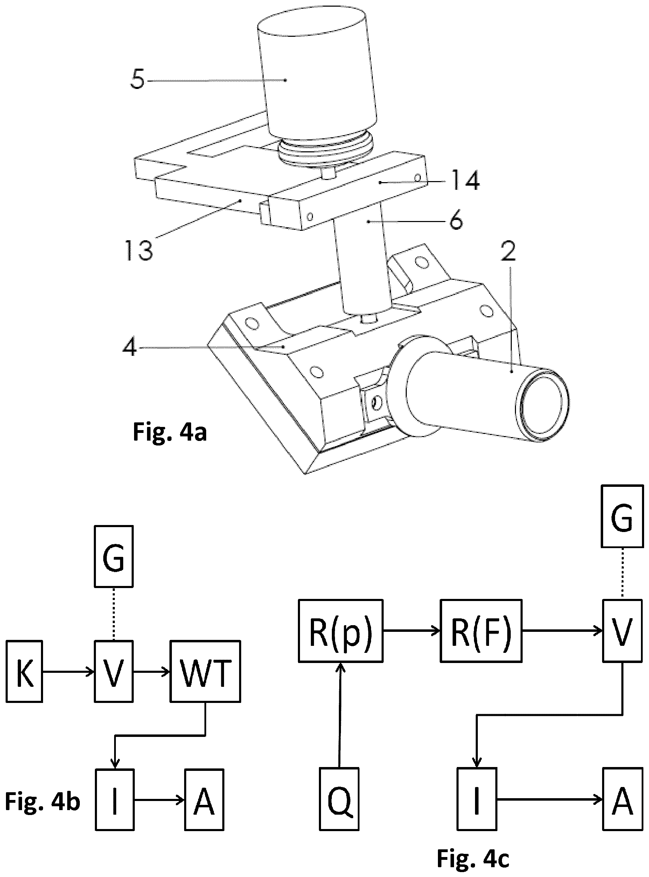

Einerseits ist eine hohe Geschwindigkeit des Treibmittels bei Eintritt in die Pulverkavität (1) vorteilhaft in Bezug auf die Dispersion des Pulvers und somit für die Inhalierbarkeit der vom Zerstäuber ausgegebenen Aerosolpartikel ist, andererseits ist es aber nicht wünschenswert, wenn der ganze Zerstäubungsvorgang innerhalb eines kleinen Bruchteils einer Sekunde abgeschlossen ist. (gemäß Beobachtungen mit einer Hochgeschwindigkeitskamera sprüht eine Treibgaskartusche mit einem 100 Mikroliter-Ventil ca. 50 bis 60 Millisekunden.) Dies würde es einem Patienten erschweren, sein Einatemverhalten mit der Erzeugung des einzuatmenden Aerosols zu koordinieren. Daher wurde hier ein Verfahren entwickelt, den Zerstäubungsvorgang in mehrere kurze Zerstäubungsvorgänge aufzuteilen und diese in einem Zeitintervall zusammenzufassen, das in seiner Dauer einem Einatemzug eines Patienten entspricht. Ein Zeitintervall, das sich gut für eine solche Koordination eignet, hat die Größenordnung von 1 Sekunde. Durch die Zerlegung der Zerstäubung in mehrere gestaffelte Vorgänge kann die Aerosol-Ausgabe insgesamt verlangsamt werden, während das Treibmittel selbst mit der für die Dispersion des Pulvers so geeigneten hohen Geschwindigkeit in der Pulverkavität (1) eintrifft.
Im Folgenden werden hierzu verschiedene Aufbauten vorgestellt, die dazu geeignet sind, mehrere aufeinanderfolgende Treibmittel-Druckstöße (Pulse) abzugeben.
Das Flussbild in Fig. 4a zeigt eine Anordnung, in der Treibmittel aus einer Kartusche K gepulst einem Zerstäuber I zur Erzeugung des Aerosols A zugeführt wird. Das flüssige Treibmittel aus dem Dosierventil der Kartusche K wird zu einem hydraulischen Magnetventil V geleitet (beispielsweise ein handelsübliches 2/2-Wege-Flipper-Magnetventil). Das Magnetventil V öffnet und schließt sich im Bereich von Millisekunden und entlässt dabei eine über die Öffnungszeit definierte Menge Treibmittel in den am Zerstäuber I angeschlossenen Verdampfer oder Wärmetauscher WT. Mittels eines handelsüblichen Pulsgenerators G, der zur Steuerung des Magnetventils V verwendet wird, werden die Dauer der Öffnungszeit, Anzahl und zeitlicher Abstand der Pulse eingestellt. In diesem Aufbau werden die Treibmittelstöße flüssig abgemessen. Ein Abmessen des bereits verdampften Treibmittels wäre theoretisch auch möglich, hat aber den Nachteil, dass bei jedem Puls das Restvolumen Gas und der daraus resultierende Druck kleiner werden.
Ein Volumen V₁ von 100 Mikroliter flüssiges Treibmittel R134a besitzt bei Raumtemperatur eine Dichte D von 1210 [kg/m³]. Anhand der molare Masse M von 0,1024 [kg/mol] ergibt sich mit der Umformung gemäß n=D*V₁/M eine Stoffmenge n von 0,00118 mol. Aus dem idealen Gasgesetz p=n*R*T/V₂ ergibt sich bei Raumtemperatur T, dem Normaldruck p und der idealen Gaskonstante R näherungsweise ein Volumen V₂ von ca. 28 Milliliter für die in den gasförmigen Zustand überführte Treibmitteldosis.
Für eine besonders gute Dispersion des Pulvers, auf das die Treibmittelpulse im Zerstäuber geleitet werden, ist es vorteilhaft, möglichst scharf begrenzte Pulse ohne große Geschwindigkeitsvariation im Treibmittel zu haben. Der Wechsel aus Abreißen der Strömung gefolgt von sehr schnellen Strömungsbeschleunigungen begünstigt die Dispersion, führt also zu einer besseren Lungengängigkeit der Aerosolpartikel bzw. zu einem höheren Feinanteil (FPD). Das Ventil zur Erzeugung der Treibmittelpulse lässt sich also bevorzugt quasi sprungartig öffnen und schließen. Insbesondere ist das Ventil so gewählt, dass die Öffnungs- oder Schließbewegung wesentlich weniger Zeit in Anspruch nehmen als für die Verzögerung zwischen zwei Pulsen. Das hydrauliche Magnetventil mit Ansteuerung durch einen Pulsgeber erwies sich hierfür als gut geeignet. Vergleichsweise deutlich weniger geeignet war eine völlig andere Anordnung (nicht dargestellt), bei der versucht wurde, die Pulsung des Treibmittels in Form von verflüssigtem Gas aus einer Kartusche (5) durch Einleitung in rotierende Kavitäten von 7 bis 23 Mikroliter Volumen (nach Kartuschen- Dosierventil mit Volumen von 100 Mikroliter) und anschließende Abgabe des Treibmittels aus den Kavitäten in das Zerstäubersystem herzustellen. Durch die Verwendung von solchen rotierenden Kavitäten (vorgesehen zwischen Kartusche (5) und Verdampfer (6), Drehung beispielsweise unterstützt durch einen Elektromotor) konnten bei Messungen zwar die Entleerung von Pulverkavitäten (1) ein wenig verbessert werden (Werteverbeserung um bis zu 10% hin zu größeren Drehzahlen im Messbereich zwischen 700 und 2000 Umdrehungen pro Minute und zu kleineren Volumina der Kavität); diese kleine Verbesserung bzgl. der Pulverausbringung ging jedoch einher mit einer deutlichen Verschlechterung der Dispersion der auszubringenden Formulierung (um 30% geringerer inhalierbarer Wirkstoffanteil). Gründe für die schlechtere Dispergierung waren dabei vermutlich zum einen, dass durch die Anordnung die sich auf die Güte der Zerstäubung auswirkende Geschwindigkeit des Treibmittels abgebremst wurde, und zum anderen, dass die Vorrichtung zur Pulserzeugung wegen der Anforderung der Drehbarkeit nicht komplett gasdicht war und somit der Treibmittelstrom zwischen den Pulsen nie ganz abbrach (die mit dieser rotierenden Anordnung erzeugten Pulse stellten also lediglich eine Oszillation eines anhaltenden Treibmittelstromes da).

Bei Verwendung einer Magnetventil-Anordnung (gemäß Fig. 4a und Fig. 4b) können die Pulse jedoch bemessen werden, ohne dass es zu permanenten Restströmungen von Treibgas oder zu einer maßgeblichen Abbremsung des Treibgases selbst kommt. Für die im Rahmen dieser Entwicklung mit der Magnetventil-Anordnung durchgeführten Messungen wurden zwischen zwei Pulsen bedingt durch die technischen Gegebenheiten des Magnetventils und seiner Ansteuerung mindestens 100 Millisekunden Verzögerung zwischen zwei Pulszeiten angesetzt. Unter der Maßgabe, die Zeit der Treibmittelausgabe auf nahezu eine ganze Sekunde zu strecken, wurde Treibmittel aus 100 Mikroliter-Dosierventilen bei Ventil-Öffnungszeiten von 7 bis 30 Millisekunden in 12 bis 5 Pulse zerlegt, Treibmittel aus 50-Mikroliter-Dosierventilen wurde bei 7 Millisekunden Ventil-Öffnungszeit in 5 Pulse zerlegt.

Fig. 4a zeigt einen analog zu Fig. 2 als Testvorrichtung ausgebildeten Zerstäuber mit einer Ansteuerung gemäß Fig. 4b. Die Kartusche (5) ist mit ihrem Ventilstamm (7) an einer entsprechenden Anschlussstelle einer Flanschplatte (14) angeschlossen. Über einen Kanal in dieser Flanschplatte (14) wird das Treibmittel aus der Kartusche einem handelsüblichen Magnetventil (13) bzw. Magnetventil-Einheit zugeleitet. Der Übergang zwischen Flanschplatte und die Magnetventil-Einheit um die Zu- und Abflüsse kann bei geeigneter Materialwahl (z.B. zweier Kunststoffe) durch festes Anpressen von Flanschplatte (14) und Magnetventil-Einheit gegeneinander oder durch zusätzliches Einfügen von Dichtelemente zwischen Flanschplatte (14) und Magnetventil-Einheit abgedichtet werden. Anschlusstelle und Kanal bis zum Magnetventil (13) sind so ausgelegt, dass ihr inneres Volumen die bei Betätigung des Ventils der Kartusche (5) abgegebene Menge Treibmittel aufnehmen kann, d.h. z.B. beträgt dieses innere Volumen mindestens 100 Mikroliter bei Verwendung eines 100-Mikroliter-Dosierventils in der Kartusche (5). Somit liegt dann nach Auslösung des Dosierventils die gesamte, für eine Dosisausbringung vorgesehene Treibmittelmenge direkt am Magnetventil (13) an. Darüber hinaus sind die Kanäle in der Flanschplatte (14) insgesamt jedoch so klein und kurz bemessen, dass das Totvolumen darin möglichst gering gehalten wird.

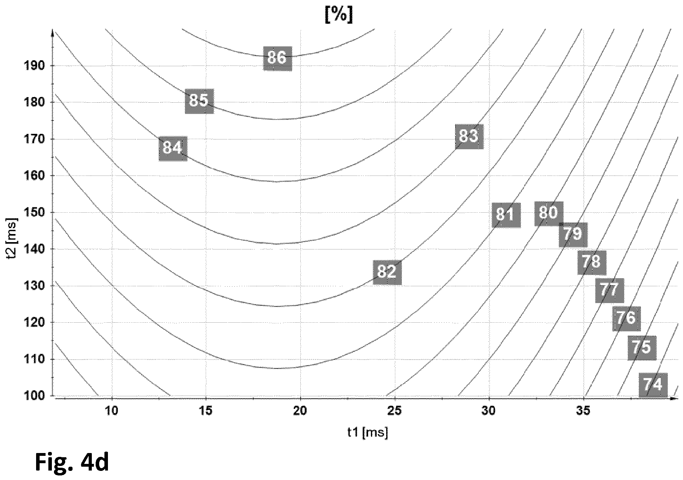

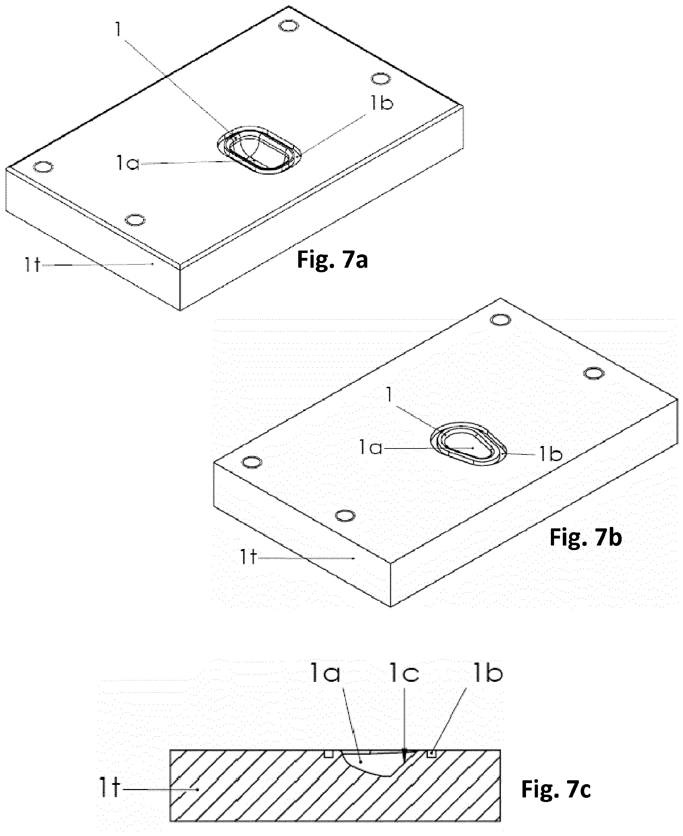

Durch geeignete Einstellungen am angeschlossenen Pulsgenerator wird die in der Flanschplatte (14) eingebrachte Treibmittelmenge dann portionsweise durch das Magnetventil (13) durchgelassen, so dass die erfindungsgemäßen Treibmittelpulse entstehen. Je kürzer die Öffnungszeiten des Magnetventils (13) sind, umso kleinere Volumina des Treibmittels werden abgetrennt bzw. portioniert und umso mehr Treibmittelpulse werden erzeugt. Bei den hier zugrunde liegenden Messungen wurde beispielsweise mit Öffnungszeiten t₁ im Bereich von 7 bis 40 Millisekunden und Verschlusszeiten t₂ im Bereich von 100 bis 200 Millisekunden gearbeitet. Das beigefügte Diagramm in Fig. 4d zeigt die Abhängigkeit der Ausbringung aus einer mit 50mg der Formulierung mit 32,5% Wirkstoff befüllten Pulverkavität als Extrapolation aus den entsprechenden Messergebnissen. Hiernach ergäbe sich eine besonders gute Ausbringung des Pulvers bei Öffnungszeiten t₁ im Bereich zwischen 13 und 24 Millisekunden, insbesondere bei höheren Verschlusszeiten t₂, insbesondere bei Verschlusszeiten t₂ oberhalb von 160 Millisekunden. Insgesamt nimmt jedoch der Einfluss der Verschlusszeit t₂ tendenziell zu kleineren Öffnungszeiten t₁ hin zu (gerade kurzen Pulsen wirken also bzgl. der Ausbringung der des Pulvers aus der Kavität vermutlich am effektivsten, wenn alle durch die einzelnen Pulse in der Kavität erzeugten Strömungen zwischen den Pulsen komplett zum Erliegen gekommen sind). An diesem Diagramm ist auch zu sehen, wie stark die Pulsung des Treibmittels die Ausbringung des Pulvers aus der Pulverkavität (1) verbessern kann: Vergleichsmessung ergaben für die gleiche Kavität (wannenförmige Kavität gemäß Fig. 7a von 3 Millimeter Tiefe), bei gleicher Befüllung und gleicher verwendeter Düse (0,5 Millimeter Durchmesser des Düsenkanals (3a)), dass die Verwendung der Treibmittelpulsung die Entleerung der Kavität von etwa 66% (ohne Pulsung) auf 86% erhöhen kann.
Die vergleichsweise höchsten Feinanteilswerte für die ausgebrachte Dosis wurden allerdings mit den kleinsten Öffnungszeiten t₁ von 7 oder 10 Millisekunden, wobei der Feinanteil zudem anscheinend kaum von der Verschlusszeit t₂ abhängt. Aus diesem Grund und weil es wegen der besseren Koordination eines Einatemvorgangs mit der Pulssequenz wünschenswert ist, die gesamte Sequenz nicht länger als 1 Sekunde werden zu lassen ist die Verwendung von Öffnungszeiten t₁ im Bereich von lediglich 7 Millisekunden und Verschlusszeiten t₂ von etwa lediglich 100 Millisekunden gut für die Anwendung des Zerstäubers als Inhalator geeignet.
Der Auslass der Magnetventil-Einheit ist wiederum mit einem anderen Kanal in der Flanschplatte (14) verbunden und dieser weitere Kanal führt zu einem Auslass, der so gestaltet ist, dass geradewegs der Verdampfer (6) an ihn angeschlossen werden kann. Bevorzugt ist der Auslass an der Flanschplatte (14) hierzu als Deckel (8) zum Verdampfer (6) gestaltet, so dass der Körper (9) des Verdampfers (6) direkt an die Flanschplatte (14) angeschlossen werden kann. Dies trägt zu einer kurzen Auslegung der Kanalwege zwischen Magnetventil (13) und Verdampfer (6) und somit zu einer Reduzierung möglicher Totvolumina bei. In seiner Ausgestaltung als Deckel (8) des Verdampfers (6) weist der Auslass an der Flanschplatte (14) einen Vorsprung mit zentraler Kanal-Öffnung auf, wobei dieser Vorsprung geradewegs oben in den Körper (8) des Verdampfers (6) passt. Für das Ausführungsbeispiel eines radial symmetrischen Verdampfers (6) gemäß Fig. 3a und 3b bedeutet dies, dass der Auslass an der Flanschplatte (14) als außen zylindrischer Vorsprung gestaltet ist. Im Innern weist dieser Auslass vorzugsweise einen Innenkonus (8d) auf, durch den das zum Verdampfer (6) geleitete Treibmittel möglichst weit über die Wärmetauschelemnete bzw. die Kugeln (11) des Verdampfers (6) verteilt wird. Im Falle des Aufbaus als Testvorrichtung, kann der Auslass der Flanschplatte (14) alternativ auch die Form eines Ventilstammes (7) haben, der zu Vergleichszwecken an beliebige Module der Testvorrichtung angeschlossen werden kann. Bei so einer Gestaltung könnte die Verwendung des ursprünglichen Deckels (8) des Verdampfers (9) natürlich nicht entfallen, sondern der Deckel (8) würde als Verbindungselement gebraucht.
Bevorzugt ist die Flanschplatte (14) aus einem gegenüber dem Treibmittel resistenten Kunststoff gefertigt, beispielsweise aus PEEK (Polyetheretherketon). Insbesondere sollte die Anschlussplatte (14) nicht aus Metall sein, damit das Treibmittel so wenig wie möglich vor Eintritt in den Verdampfer (6) verdampft bzw. in flüssiger Phase das Magnetventil (13) durchläuft.

Fig. 4c zeigt ein Flussbild zu den Abläufen eines mit Treibgas unterstützten Zerstäubers. Alternativ zur Anbringung einer mit Treibmittel in flüssiger Form gefüllten Kartusche (5) wird in dieser Ausführungsform ein Zerstäuber mit gasförmigem Treibmittel betrieben. Prinzipiell kann gasförmiges Treibmittel wie Druckluft oder Stickstoff direkt in eine Pulverkavität geleitet werden, da es keine flüssigen Bestandteile enthält. Das Einsetzen eines Verdampfers ist dann nicht nötig. In der Regel sind Anordnungen mit angeschlossenen Druckgasleitungen und/oder angeschlossenen Gasflaschen allerdings so umfangreich, dass sie sich nur für den Einsatz als Standgerät z.B. in einem Labor nicht aber für portable Handgeräte eignen. Dieses System ist beispielsweise insbesondere für die Durchführung von Versuchen zum Einfluss der Variation von Faktoren wie Pulsanzahl, Verschlusszeit und Druck geeignet.
In Fig. 4c ist schematisch skizziert, wie ein Treibgas aus einer Quelle Q (beispielsweise eine laborübliche Stickstoff- oder Druckluftleitung oder eine Gasflasche) über einen Druckregler R(p) und einen Strömungsregler R(F) zu einem pneumatischen Magnetventil V geleitet werden. Mit dem Strömungsregler R(F) wird auch bei unterschiedlichen vorgegebenen Drücken eine Strömung konstanter Stärke eingestellt. Der Luftstrom wird dabei mit einem handelsüblichen Flussmessgerät gemessen. Das darauffolgende Magnetventil V öffnet und schließt sich im Bereich von Millisekunden und entlässt dabei eine über die Öffnungszeit definierte Menge Stickstoff in den Zerstäuber I, aus dem folglich das Aerosol A abgegeben wird. Mittels eines Pulsgenerators G, der zur Steuerung des Magnetventils V verwendet wird, werden die Dauer der Öffnungszeit, Anzahl und zeitlicher Abstand der Pulse eingestellt.
Bei geöffnetem Magnetventil V der Anordnung gemäß Fig. 4a treten 100 Mikroliter flüssiges Treibmittel aus einer Kartusche in ca. 50-60 Millisekunden aus (belegt durch Aufnahmen mit einer Hochgeschwindigkeitskamera). Zur besseren Vergleichbarkeit von Messergebnissen kann man auch bei Verwendung einer Quelle Q das gleiche Gasvolumen wie bei einem Kartuschen-Treibgasstoß mit 100 Mikrolitern pro Auslösung und mittels Pulsen eine auf bis zu 1 Sekunde verlängerte Sprühzeit nachstellen und hat dabei z.B. folgende Möglichkeiten, die Pulsstöße für das zuvor berechnete Treibmittelvolumen von 28 Millilitern einzustellen:

| Anzahl Pulse | Öffnungszeit des Magnetventils in Millisekunden | Maximale Verzögerung zwischen den Pulsen in Millisekunden |
|---|---|---|
| 1 | 50 | - |
| 2 | 25 | 500 |
| 3 | 16,7 | 330 |
| 4 | 12,7 | 200 |
| 8 | 6,25 | 125 |
| 9 | 5,2 | 110 |
| 16 | 3,125 | 60 |

Für die im Rahmen dieser Entwicklung durchgeführten Messungen wurden zwischen zwei Pulsen bevorzugt mindestens 50 Millisekunden Verzögerung angesetzt, d.h. eine Verschlusszeit t₂ von 50 Millisekunden zwischen zwei Öffnungszeiten t₁ des Ventils. Bei den Messungen wurde die Zahl der Pulse im Bereich von 1 bis 16 und die Verschlusszeit t₂ im Bereich von 50 bis 400 Millisekunden variiert. Dabei zeigte sich ein Trend zur besseren Entleerung der Pulverkavität (1) mit steigender Anzahl der Pulse (hier verbunden mit kürzeren Öffnungszeiten) und wachsender Verschlusszeit zwischen den Pulsen. Gute Ergebnisse hinsichtlich der Ausbringung von Pulver aus der Pulverkavität ergaben sich entsprechend bei jeweils mittlerer Einstellung - extrapoliert insbesondere für den Bereich von 7-10 Pulsen bei 200 bis 100 Millisekunden Verzögerung. Diese Pulszahl entspricht bezogen auf die anhand von Fig. 4a vorgestellte Anordnung Öffnungszeiten t₁ von 5 bis 7 Millisekunden.

Versuche mit Druckvariationen des Treibgases im Bereich zwischen 2 und 6 bar und Variationen der Pulszahlen im Bereich von 1 bis 16 zeigten anhand einer Pulverkavität (1) mit 0,19 Milliliter Innenvolumen (entsprechend 45 Milligramm Laktose oder 50 Milligramm der Laktose-basierten Formulierung mit 32,5% Wirkstoff), dass sich sowohl die Ausbringung des Pulvers aus der Pulverkavität (1) als auch der Feinanteil der ausgebrachten Partikel mit steigendem Druck erhöht. Der Effekt des Drucks insbesondere auf den Feinanteil nimmt dabei zunächst bei steigender Anzahl der Pulse zu. Für den Bereich einer Pulszahl von 7-14 (entsprechen Öffnungszeiten von 7 bis 4 Millisekunden) zeigten die Versuchsergebnisse bei höheren Drücken die höchsten Werte. In diesem Pulsbereich konnten vermutlich in der Pulverkavität Druckspitzen ankommenden, die besonders günstig für die Deagglomeration der Partikel sind. Bei größeren Pulszahlen scheint der Einfluss des Drucks wieder abzunehmen (vermutlich sind dann die Ventilöffnungszeiten zu kurz, als dass die volle Druckhöhe von vor dem Ventil sich hinter dem Ventil wieder aufbauen kann).

Insgesamt zeigten die Aerosolmessergebnisse, die unter Verwendung der vorgestellten Zerstäuber-Vorrichtungen mit gepulster Treibmittelzuführung erzielt wurden, dass durch die Verwendung einer hohen Anzahl von Pulsen und lange Verzögerungszeiten zwischen den Pulsen die Wirkstoffausbringung und der Feinanteil der ausgebrachten Dosis erhöht werden kann. Vergleichsmessungen zeigten eine Erhöhung des Feinanteils auf einen Wert entsprechend etwa 130% des mit einer entsprechenden Vorrichtung ohne Magnetventil erzeugten Wertes.

Insgesamt konnten bei den dieser Schrift zugrunde liegenden Messungen bei Verwendung von sowohl Magnetventil (13) zur Treibmittelpulsung als auch Verdampfer (6) sehr hohe Mengen an pulverförmigen Formulierungen aus Pulverkavitäten (1) ausgebracht werden: So war es beispielsweise möglich, von 75 Milligramm einer Pulvermischung mit 98% Fenoterol 16,5 Milligramm Fenotorol tatsächlich als Feinanteil zu gewinnen (bei einer Entleerung der Pulverkavität (1) von 92,5%, bei einer angelegten Flussrate von 30 Litern pro Minute). Bei Verwendung einer solchen Treibmittel-getriebenen Vorrichtung mit Verdampfer und Magnetventil schien im Rahmen der hier durchgeführten Messungen die Stärke der am Mundstück des Zerstäubers angelegten Flussrate keinen signifikanten Einfluss auf den Feinanteil des ausgebrachten Dosis mehr zu haben (geteste Flussraten variierten im Bereich von 30 bis 90 Litern pro Minute).

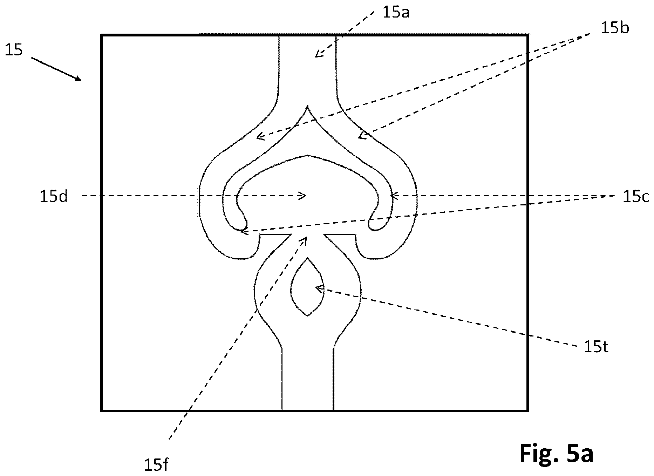

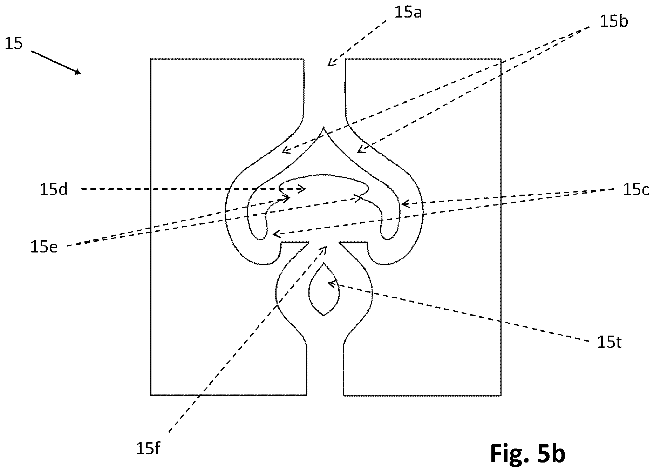

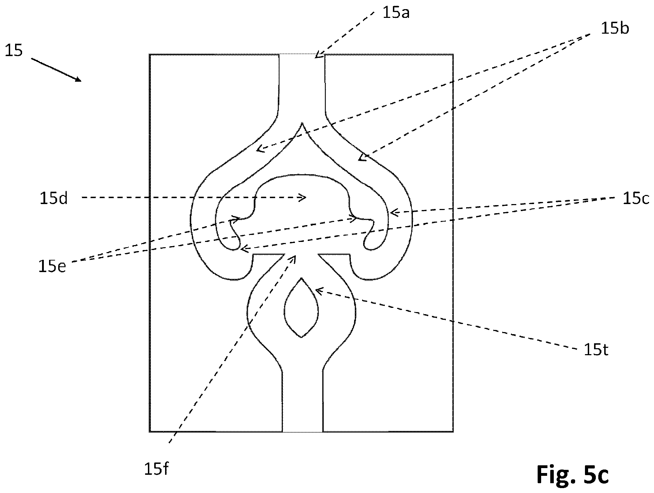

In Fig. 5a, b und c sind verschiedene Kanalstrukturen zu sehen, die jeweils einen mikrofluidischen Oszillator (15) bilden, wie er insbesondere alternativ zum anhand von Fig. 4 beschriebenen Magnetventil (13) in die Treibmittelzuführung eines Geräts zur Zerstäubung eingefügt werden kann. Vorzugsweise befindet sich dieser mikrofluidische Oszillator (15) im Strömungsverlauf ebenfalls zwischen der mit Treibmittel bzw. flüssigem Treibgas befüllten Kartusche (5) mit Dosierventil und dem der Einleitung in die Pulverkavität (1) vorgeschalteten Verdampfer (6).

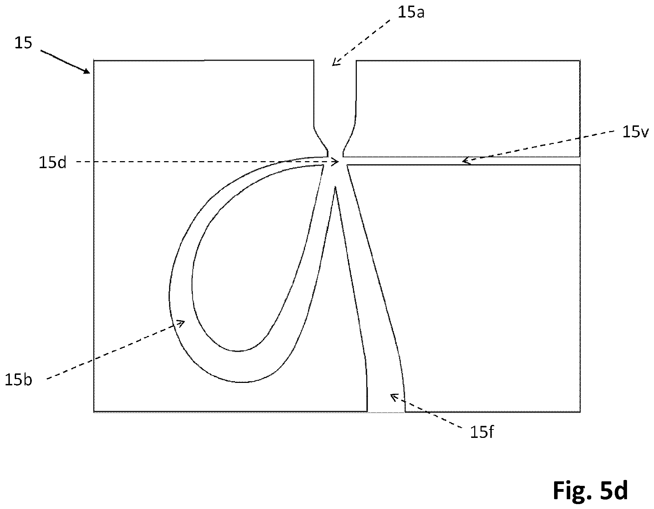

Der Strömungsverlauf in Fig. 5 a-d ist von oben nach unten abgebildet. In einer Einbausituation wird der Ausgang des Dosierventils der Kartusche (5) an den Einlasskanal (15a) der Kanalstruktur angeschlossen. In den Ausführungsformen des mikrofluidischen Oszillators (15) nach Fig. 5a, 5b und 5c gabelt sich der Einlasskanal (15a) an einer Gabelung in zwei Teilkanäle (15b) auf. Die Gabelung ist dabei bevorzugt so gestaltet, dass sich die Flüssigkeit möglichst reibungsarm an die (auf die Kanalstruktur insgesamt bezogen) jeweils inneren Wände (15c) der Teilkanäle (15b) anschmiegen kann. Der Beginn der Gabelung kann hierzu beispielsweise V-Förmig (wie in Fig. 5a, 5b und 5c zu sehen) oder kreisbogenförmig ausgelegt sein, insbesondere ist die Gabelung dabei symmetrisch zu der durch den Einlasskanal (15a) gebildeten Achse ausgebildet. Die gesamte Kanalstruktur weist bevorzugt eine Spiegelsymmetrie um die durch den Einlasskanal (15a) gebildete Achse auf. Im weiteren Verlauf krümmen sich die Teilkanäle (15b) nach innen und münden in einen Mischbereich (15d) ein. Dabei werden sie entsprechend des Coanda-Effekts bevorzugt immer an der jeweils inneren Wand entlang geführt. Bevorzugt wird die Strömung zusätzlich nach Eintritt in den Mischbereich (15d) von den entsprechenden Innenwänden (durch geeignet gewählte Krümmungen der Wände) zumindest ein Stückchen entgegen der insgesamt durch Ein- und Auslass der Kanalstruktur vorgegebenen Hauptströmungsrichtung umgelenkt. In der in Fig. 5a gezeigten Ausführungsform werden die Flüssigkeitsströmungen unter Umständen bis zum oberen Rand des Mischbereichs (15d) zurückgeführt, wo die Strömungen aus den beiden Teilkanälen (15b) dann spätestens wieder aufeinander treffen. In der Ausführungsform nach Fig. 5b und 5c werden die Fluidstrahlen aus den beiden Teilkanälen (15b) durch Vorsprünge (15e), die sich an die jeweiligen Strömungsführungsabschnitte der Wand des Mischbereichs anschließen, mitten in den Mischbereich (15d) aufeinander zu geleitet. Ziel dieser Umlenkungen ist es, möglichst viele Wirbel im Mischbereich (15d) zu erzeugen. Durch diese Wirbel kommt der Abfluss des Fluids durch die Öffnung des Auslasses (15f) aus dem Mischbereich (15d), der hier auch als Oszillationskammer bezeichnet werden kann, immer ins Stocken, wobei abwechselnd den jeweiligen Strömungen aus den beiden Teilkanälen (15b) der Vorrang gegeben wird, worauf es dazwischen auch zum Abbrechen der Strömung kommt. Dieses Verhalten war in Strömungssimulationen gut zu erkennen. Durch den Betrieb mit verflüssigtem Treibgas wird das "Stocken" durch Gasblasenbildung im Mischbereich (15d) verstärkt. Bei den Verwirbelungen entstehen im Mischbereich (15d) Stellen mit unterschiedlichen Dichten im Fluid, so dass es zu Siedeverzügen und Gasblasenbildung kommt. Diese Gasblasen tragen dann ihrerseits noch zu einem schubartigen Austrieb des Fluids aus dem Mischbereich (15d) bei.
Optional (aber nicht notwendigerweise) kann anfangs in dem an den Auslass (15f) angeschlossenen Kanal noch ein Strömungsteiler vorgesehen sein, der gemäß dem Coanda-Effekt abwechselnd mal eine Strömung nach links für die Strömung aus dem ursprünglich rechten Teilkanal (15b) und mal eine Strömung nach rechts für die Strömung aus dem ursprünglich linken teilkanal (15b) begünstigt.

Fig. 5d zeigt einen alternativen mikrofluidischen Oszillator (15). In diesem Ausführungsbeispiel führt der Einlasskanal (15a) auf eine Erweiterung, in die von den Seiten ein Steuerungs- oder Lüftungskanal (15v) einmünden kann. An den Erweiterungsbereich schließt sich eine Kanalgabelung an, wobei diese Gabelung in einen Teilkanal (15b) und einen Auslass (15f) aufgeht. Der Teilkanal (15b) ist dabei so gestaltet, dass er das durch ihn gelangende Fluid wieder seitlich in den Erweiterungsbereich (sozusagen als Zuführungskanal) zurückführt. Der Erweiterungsbereich kann somit auch als Mischbereich (15d) bezeichnet werden. Die Symmetrie von Einlasskanal (15a) und Gabelung ist wie in den beiden vorherigen Ausführungsbeispielen wiederum so, dass gemäß dem Coanda-Effekt eine Strömung in beide Zweige der Gabel gleichermaßen begünstigt sind. Durch ein Druckgefälle, das beispielsweise seitlich über einen Lüftungskanal (15v) anliegt, kann die Strömung beeinflusst werden beispielsweise zunächst in den Teilkanal (15b) zu strömen. Das erneute Austreten dieses Fluidstrahls als Zuführung in den Erweiterungsbereich kann dann die Vorzugsrichtung des Strahls in Richtung Auslass (15f) lenken, so dass kurzzeitig Fluid das Bauteil verlässt, aber verursacht durch den Lüftungskanal kann die Vorzugsrichtung wieder in Richtung Teilkanal (15b) umschlagen, so dass sich ein oszillierendes System ergibt.

Über die Darstellungen von mikrofluidischen Bauteilen in Fig. 5a-d hinaus ist es auch möglich, Kanalstrukturen mit mikrofluidischem Oszillator vorzusehen, die beispielsweise am Auslass des Mischbereichs so ausgelegt sind, dass es zur Verdampfung des bis dahin noch nicht gasförmigen Anteil des Treibmittels kommt, so dass ein sich als zusätzliches Bauteil im Strömungsverlauf anschließender Verdampfer (6) entfallen kann, oder direkt in der die Kanalstruktur bildenden Einheit mit ausgebildet ist.
Möglichkeiten, die geschilderten Kanalstrukturen zu fertigen, bieten sich beispeilsweise durch Silizium-Ätztechniken, LIGA-Verfahren oder durch sonstige Verfahren zur Erzeugung von Mikrostrukturen, insbesondere mikrofluidischen Systemen. Abgestimmt auf solche Herstellungsverfahren ist die hier dargestellte Kanalstruktur vorzugsweise zweidimensional, d.h. sie besteht bevorzugt aus einer Platte, in der die Kanäle beispielsweise mit rechteckigem Querschnitt ausgebildet sind, wobei ein Deckel auf der Platte fixiert ist und so die Kanäle längsseitig verschließt.

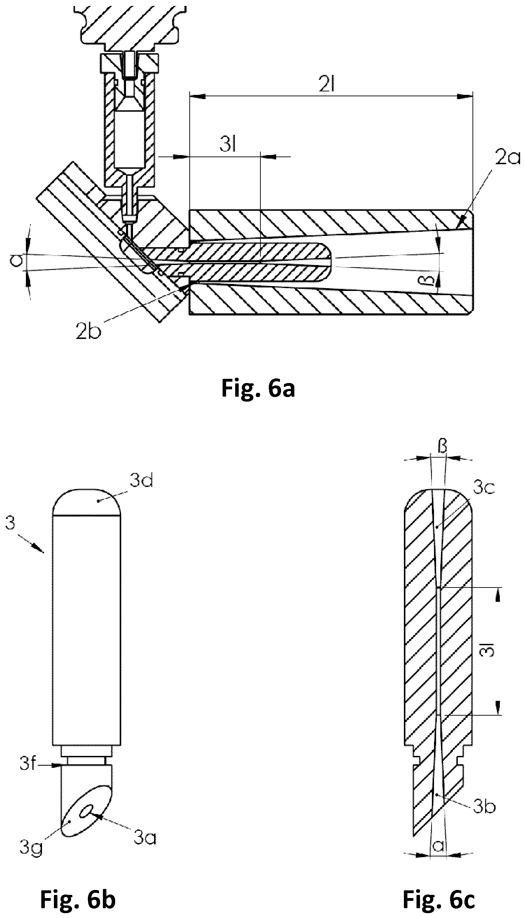

In Fig. 1a ist zu sehen, wie ein Zuführkanal im Anschlussstück (4) geradewegs zur die Pulverkavität (1) führt und wie der Düsenkanal der Düse (3), die anhand von Fig. 6 a-c später genauer beschrieben wird, direkt von der Pulverkavität (1) abgeht. Für diese Anordnung bevorzugte Pulverkavitäts-Formen werden in Fig. 7a und in Fig. 7b und Fig. 7c gezeigt. Fig. 7a zeigt einen speziell für die Verwendung in einer Testvorrichtung entsprechend Fig. 2 aufgebauten Träger (1t), der die Pulverkavität (1) beinhaltet. Insbesondere für die Verwendung in einer in einer Testvorrichtung besteht der Träger (1t) oder zumindest der Bereich, der die Pulverkavität (1) bildet, vorzugsweise aus einem durchsichtigen Material wie beispielsweise PMMA, so dass die Entleerung der Pulverkavität (1) während der Zerstäubung bzw. während der Zuleitung von Treibmittel beispielsweise mit einer Kamera beobachtet werden kann. Die Pulverkavität (1) weist einen - hier in der Abbildung - wannenförmigen Napf (1a) für die Aufnahme der medizinischen Formulierung bzw. des Pulvers auf. Die Öffnung des Napfs (1a) ist an der Oberseite des Trägers (1t) von einer Dichtungsnut (1b) umgeben, in die ein Dichtungsmaterial wie beispielsweise ein vorzugsweiser elastomerer O-Ring eingelegt wird, der beim Zusammenbau von Träger (1t) und Anaschlussstück (4) das Innere der Pulverkavität (1) im Bereich zwischen Träger (1t) und Anschlussstück (4) umlaufend abdichtet. Die Öffnung des Napf s (1a) in Fig. 7a weist einen nahezu rechteckigen Querschnitt mit abgerundeten Ecken oder einen ovalen Querschnitt (nicht dargestellt) auf. Seine Länge ist in Strömungsrichtung größer als seine Breite, vorzugsweise ist er etwa doppelt so lang wie breit. Der Boden ist vorzugsweise abgerundet und/oder in den Wandbereichen zylindrisch gewölbt, so dass die Pulverkavität (1) im Napf (1a) keine Ecken aufweist, in denen sich Pulver festsetzen könnte. Bei Messungen mit der zuvor beschriebenen Testvorrichtung wurden hinsichtlich ihrer Tiefe verschieden große Näpfe (1a) verwendet: Bei gleichen Öffnungsquerschnitten wurden angepasst auf die jeweils für die Zerstäubung bereitgestellten Pulvermengen pro Pulverkavität (1), Napftiefen von 1, 2, 3 und 4 Millimetern getestet. In die entsprechenden Näpfe (1a) passten jeweils 15, 30, 45 und 70 Milligramm Laktose (bei beispielsweise 190 Mikroliter Volumen für den 3 Millimeter tiefen Napf (1a)). Bei den Testmessungen ergab sich ein besseres Entleerungsverhalten zu flacheren Näpfen (1a) hin: Bei Befüllung mit Laktose konnten für die Tiefen von 1, 2, 3 Millimeter ohne Pulsung des Treibmittels Entleerungsgrade von bis zu 85%, 80% und 70% erzielt werden. Im zusammengebauten Zustand mündet ein Zuführkanal aus dem Anschlussstück (4) in der Nähe eines Endes des wannenförmigen Napfes (1a) und der Düsenkanal (3a) schließt sich in der Nähe des anderen Endes des Napfes (1a) an. Fig. 7b und Fig. 7c zeigen eine alternative, bevorzugte Ausführungsform der Pulverkavität (1). Diese Ausführungsform weist in vielen Dingen die gleichen Merkmale (inklusive ihrer Anschlüsse) wie die anhand von Fig. 7a beschriebene Ausführungsform auf, doch weicht sie insofern davon ab, als dass der Napf (1a) nur noch in Querrichtung und nicht mehr in Längsrichtung symmetrisch ist. Die Pulverkavität (1) weist eine so genannte Tropfenform auf. Der "Bauch" des Tropfens befindet sich dabei in der Nähe des Zuführungskanals und das schmale Ende auf der Seite, wo sich der Düsenkanal (3a) anschließt. Vorzugsweise weist nicht nur die Öffnung des Napfs (1a), sondern auch sein Boden die Tropfenform auf. Besonders bevorzugt weist der Boden des Napfes (1a) einen ein wenig aus der Längsmitte in Strömungsrichtung verschobenen Tiefpunkt auf, der optional als kleine Abflachung (1d) gestaltet ist, und an diesen Tiefpunkt schließt sich in Strömungsrichtung eine Schräge (1c) an. Diese Schräge (1c) verläuft dabei steiler als der Bodenverlauf des Napfes (1a) in Strömungsrichtung gesehen vor dem Tiefpunkt war. Auf diese Weise wird die Strömung bereits im Napf (1a) auf den sich anschließenden Düsenkanal (3a) ausgerichtet: Durch das spitze Zulaufen der Tropfenform wird die Pulverbeladene Strömung einerseits in ihrem Querschnitt dem Eingangsquerschnitt am Düsenkanal (3a) angenähert und andererseits durch die Schräge gezielter in die Richtung des Düsenkanals (3a) geschickt. Vorzugsweise bildet die Schräge (1c) hierbei in Relation zur Oberfläche des Trägers (1t) den gleichen Winkel wie die Achse des angeschlossenen Düsenkanal (3a), bzw. vorzugsweise setzt sich die Richtung der Schräge (1c) in der des Düsenkanals (3a) fort. Bei Testmessungen mit Laktose zeigten solche tropfenförmigen Pulverkavitäten (1) höhere Entleerungsgrade als die vergleichsweise linearen, wannenförmigen Pulverkavitäten (1) (wie sie anhand von Fig. 7a beschrieben wurden). Für bis zu 3 mm tiefe, tropfenförmige Pulverkavitäten (1) wurden bereits selbst ohne Pulsung der Treibmittelstöße Entleerungsgrade zwischen 95% und 100% erzielt (beispielsweise 99,4% gemessen mit Düsenquerschnitten von 50 Millimetern). Dabei wurden in der Regel Näpfe (1a) mit einer Tiefe von 3 Millimetern an der Abflachung (1d) und einem inneren Volumen von 50 Mikrolitern (entsprechen der Aufnahme von 21 Milligramm Laktose) verwendet. Angepasst an die jeweils für die Zerstäubung vorgesehene Pulvermenge, können die Tiefen der tropfenförmigen Näpfe (1a) analog zu denen der wannenförmigen Näpfe (1a) beispielsweise im Bereich zwischen 1 und 5 Millimeter Tiefe oder mehr verändert werden, nehmen aufgrund ihrer speziellen Geometrie jedoch bei gleicher maximaler Tiefe weniger Pulver als die wannenförmigen Näpfe (1a) auf.

Fig. 6b und Fig. 6c zeigen die Düse (3) im Detail. Sie weist einen zentralen Düsenkanal (3a) auf, der sich zu Beginn in einen Einlasskonus (3b) mit einem Einlasswinkel a und zum Ende hin in einen Auslasskonus (3c) mit Auslasswinkel β öffnet, a und β betragen bevorzugt beide 5°, aber auch andere, von einander abweichende Winkelgrößen sind möglich. Zwischen diesen beiden Konussen weist der Düsenkanal (3a) einen so genannten zylindrischen Bereich auf, in dem der Querschnitt über eine definierte Länge (3l) konstant ist.
Die Anschlussseite (3g) mit der die Düse (3) in der dargestellten Ausführungsform der Testvorrichtung an die Pulverkavität (1) angelegt wird, ist entsprechend der Geometrien des Anschlussstücks (4) gegenüber der durch den Düsenkanal (3a) gebildeten Achse abgewinkelt, hier vorzugsweise um 45°. Bei diesem Winkel trifft zuvor auch das Treibmittel in einem 45° Winkel auf die Pulverkavität (1) aber auch andere insbesondere flachere Winkel sind möglich. Vorteilhaft ist es, hierbei den Abgang des Düsenkanals (3a) von der Pulverkavität (1) spiegelbildlich im gleichen Winkel wie die Zuleitung des Treibmittels auszulegen. Diese spiegelbildliche Auslegung ist hier abgestimmt auf die an die Pulverkavität (1) angeschlossene Düse (3) und ihren speziellen geradlinigen Aufbau. Bei der Wahl einer anderen Düse (3) wie beispielsweise einer Wirbeldüse oder einer Düse, deren Funktion eine vorangegangene Wirbelkammer bedingt, liegt diese geradlinige Anordnung unter Umständen nicht vor. Bei Verwendung einer Düse mit Wirbelkammer (nicht in den Abbildungen dargestellt) ist es abweichend von dem oben Gesagten zweckdienlich, wenn die Pulverkavität (1) selbst eine Wirbelkammer bildet, also z.B. einen kreisförmigen Durchmesser, vorzugsweise mit flachem Boden hat und der Treibmittelstrom nahe des Randes der Pulverkavität (1) eintrifft. Der Auslass der Düse liegt dann mittig über der Pulverkavität (1). Versuche mit solchen Wirbelkammern wurden auf diese Weise ebenfalls mit der hier geschilderten Testvorrichtung durchgeführt, lieferten aber bei den hier gemessenen vergleichweise großen Pulvermengen aufgrund von hohen Rückständen in den Pulverkavitäten schlechtere Zerstäubungsergebnisse (lediglich Entleerungsgrade von 60%) als die anhand von Fig. 6 detaillierter beschriebenen Düsenanordnungen in Verbindung mit den anhand von Fig. 7 beschriebenen Pulverkavitäten (1).

Nach Austritt aus der Düse (3) wird das Pulver von Luftströmung im Mundstück (2) mitgerissen. Bei Labormessungen mit der Testvorrichtung wird dieser Luftstrom, der beim Inhalator durch Einatmung des Patienten erzeugt wird, durch ein System zur Strömungserzeugung nachgestellt. In beiden Fällen kommt eine Luftströmung im Mundstück (2) durch einen Luftsog am Ende des Mundstück (2) dadurch zustande, dass Luft an einer entgegen gesetzten Stelle in das Mundstück (2) oder in das Gerät durch mindestens eine Einlassöffnung (2b) eintreten kann. Bevorzugt weist der Inhalator bzw. die Testvorrichtung im Bereich von Düse (3) und Mundstück (2) ein oder mehrere mit Einlassöffnungen (2b) verbundene Kanäle auf, die so im Mundstück (2) einmünden, dass die durch die Kanäle strömende Luft den aus der Düse (3) austretenden Treibmittelstrom einhüllt und so die Bestandteile in besonders geeigneter Weise mitnimmt. Insbesondere bilden diese Kanäle einen Bypass zur Düse. Der aerodynamische Durchmesser dieses Bypasses - insbesondere an dessen engster Stelle - bestimmt hierbei den Einatemwiderstand, den ein Patienten beim Inhalieren aus einem analog aufgebauten Inhalator verspürt.
Mit der Testvorrichtung wurden Messungen durchgeführt, in denen ein Luftsog mit Flussraten von 30, 60 und 90 Liter pro Minute am Mundstück (2) angelegt und somit ein unterschiedliches Einatemverhalten von Patienten nachgestellt wurde. (30 Liter pro Minute gelten hierbei gemäß der Anweisungen im Europäischen Arzneibuch für die aerodynamische Beurteilung von Dosieraerosolen, 90 Liter pro Minute entsprechen einem Unterdruck von 4 Kilopascal bei einem passiven Pulverinhalator.) Die FPD-Ergebnisse belegten, dass für dieses Zerstäubungskonzept (Ausbringung von trockenem Pulver durch einen länglichen, geradlinigen Düsenkanal mittels in einem Verdampfer getrocknetem Treibmittel) keine nennenswerte Abhängigkeit der Zerstäubung vom Einatemverhalten besteht (auch bereits ohne Pulsung des Treibmittels). Bezogen auf die insgesamt aus dem Zerstäuber ausgebrachte Wirkstoffmenge wurden bei der Flussrate von 30 Litern pro Minute etwas stärkere Ablagerungen im Eingangsbereich des angeschlossenen Messgeräts festgestellt (dieser Eingangsbereich korrespondiert unter Einschränkungen mit dem Mund- und Rachenraum eines Patienten, wenn man diese Messergebnisse auf die Anwendung eines Inhalators überträgt). Bei den Flussraten von 60 und 90 Litern pro Minute zeigte das Zerstäubungsverhalten hinsichtlich der insgesamt ausgebrachten Wirkstoffmenge keinen signifikanten Unterschied. Diese höheren Flussraten sind offensichtlich besser geeignet, auch die größeren Partikel des Aerosols mitzureißen. Insgesamt zeigen die Zerstäubungsdaten für das hier vorgestellte Konzept eine deutlich geringere Flussraten-Abhängigkeit als die meisten kommerziell erhältlichen Pulverinhalatoren.

Bevorzugt ist, wie auch in Fig. 6a zu sehen, die Düse (3) bzw. das sie bildende Modul in eine Durchführung (2a) am Mundstück (2) eingeschoben und die Kanäle befinden sich zwischen dem Modul, das die Düse (3) bildet, und einer inneren Wand des Mundstücks (2). Besonders bevorzugt sind Düse und Mundstück im Wesentlichen radialsymmetrisch gestaltet und so zueinander angeordnet, dass ein im Wesentlichen ringförmiger Kanal (der allenfalls durch Halterungselemente zwischen Düse (3) und Mundstück (2) unterbrochen ist) zwischen Düse (3) und Mundstück (2) vorliegt. Optional verjüngt sich die Ringstärke des Kanals zur Einlassöffnung (2b) hin. Dies wird bei einer nicht dargestellten Ausführungsform noch verstärkt, in der die Düse ebenfalls außen eine Konusform aufweist, wobei die Konusneigung der Düse (3) entgegengesetzt zur Konusneigung der Durchführung (2a) am Mundstück (2) ausgebildet ist (d.h. die breiteste Stelle des die Düse (3) bildenden Bauteils sitzt an der schmalsten Stelle der Durchführung (2a)). Bevorzugt hat die Durchführung (2a) am Mundstück (2) eine Konusform, wobei sie sich insbesondere mit einem Winkel von 0° bis 35° und besonders bevorzugt mit einem Winkel von 0° bis 15° wie beispielsweise 5° zu dem Ende des Mundstücks (2) hin öffnet, an dem bei Verwendung bzw. im Betrieb der Sog angelegt wird. Messungen des Feinanteils (FPD) ergaben, dass sich der Feinanteil bei Verwendung der kleineren Konuswinkeln im Mundstück (im Vergleich zur Verwendung der größeren) erhöht, d.h. das die Lungengängigkeit der zerstäubten Partikel besser wird.
Bevorzugt ist die Öffnung des Mundrohrs (2) an der Stelle des Auslass der Düse deutlich größer als die Öffnung des Düsen-Auslasses, bevorzugt mindestens 5 mal so groß bezogen auf die Durchmesser. Dies begünstigt die Aerodynamik im Mundrohr.
Bevorzugt ist das Mundstück (2) an besagtem Ende länger als die eingeschobene Düse (3). Bei variierenden Längen (2l) des Mundstücks (2) von insgesamt 15 bis 120 Millimetern wurde (bei festgehaltener kürzerer Länge der Düse (3) von vorzugsweise maximal 15 Millimetern) festgestellt, dass bei den kürzeren Mundstücken zwar geringere Formulierungs-Ablagerungen an der Wand der Durchführung (2a) auftraten, dafür jedoch bei den längeren Mundstücken die Zerstäubungswolke eine bessere Aerodynamik aufwies. Bei den längeren Mundstücken (2) (insbesondere bei denen mit einer Länge (2l) von 120 Millimetern) zeigten Messungen eine Erhöhung des Feinanteils (FPD). Vermutlich kommt es durch die Strömung im Mundrohr zu einem günstigen Abbremsen der durch das Treibgas beschleunigten Partikel, so dass sich bei Anwendung eines solchen Zerstäubers die mögliche Deposition dieser Partikel in der Lunge des Patienten erhöht.
Bei diesen beiden gegenläufigen Effekten (Bildung von Ablagerungen innen im Mundstück (2) und Erhöhung des Feinanteils im Aerosol) ergibt sich ein bevorzugter Längenbereich für das Mundstück (2), nämlich 30 bis 90 Millimeter, besonders bevorzugt 60 bis 90 Millimeter, oder einen Überstand des Mundstücks (2) gegenüber dem Ende der Düse (3) von 20 bis 70 Millimeter, besonders bevorzugt von 40 Millimeter.

Die Düse (3) wird im Ausführungsbeispiel der Testvorrichtung so ins Anschlussstück (4) eingesetzt, dass sie mittels einer in die Sicke (3f) eingesetzten Dichtung derart abgedichtet wird, dass es nicht zu Bypass-Strömungen außen an der Düse (3) in die Pulverkavität (1) kommt.
Die Länge des Düsenkanals und die Länge (3l) des zentralen zylindrischen Teils des Düsenkanals (3a) sind wichtige Funktionsparameter der Düse (3) wie sie in Fig. 1a und Fig. 6a-c gezeigt wird. Ebenso wie die Länge (2l) des Mundstücks (2) wurde die Länge des Düsenkanals (3a) im Rahmen von Messungen mit der Testvorrichtung von 3 Millimetern bis 15 Millimetern variiert. Auch hier ergab es sich bei Messungen des Feinanteils (FPD), dass sich der Feinanteil bei Verwendung der größeren Längen (insbesondere bei 15 Millimetern) erhöht, d.h. das die Lungengängigkeit der zerstäubten Partikel bei der Verwendung längerer Düsen (3) besser wird. Dies erklärt sich durch die längere Einwirkzeit von Scherkräften auf den Gasstrom bzw. das Aerosol in den längeren Düsenkanälen (3a). Die Einwirkzeit ist insgesamt wegen der hohen Geschwindigkeit des Gasstroms sehr kurz. Fluss-Simulationen zeigten, dass die Geschwindigkeit des Gasstroms in der Düse (3) Werte von bis zu 1 Mach annehmen kann.
Die Geschwindigkeit in der Düse ist abhängig vom aerodynamischen Querschnitt der Düse (3). Bei kleinerem Querschnitt der Düsenkanals (3a) erhöht sich der Strömungswiderstand der Düse, die Deagglomeration der Formulierungspartikel nimmt zu. Dies wurde anhand der Ergebnisse von Messungen (ohne Treibmittelpulsung) mit unterschiedlichen Querschnitten von Düsenkanälen (3a) belegt. Getestet wurden Querschnitte im Bereich von 0,2 bis 0,8 Quadratmillimetern (z.B. Variation eines kreisförmiger Durchmessers im Bereich von 0,5 bis 1 Millimeter) - bevorzugt werden hier als Resultat aus den Messungen Querschnitte im Bereich von 0,4 bis 0,7 Quadratmillimetern für den Düsenkanal (3a). Ausgewertet wurde für diese Messungen insbesondere das Verhältnis von Feinanteil (FPD) zu insgesamt aus dem Zerstäuber ausgebrachter Wirkstoffmenge. Dieses Verhältnis erhöhte sich bei kleiner werdendem Querschnitt. Durch die Verwendung kleinerer Durchmesser für den Düsenkanal (3a) und somit kleinerer Querschnittsflächen kann ein höheres Scheergefälle erzielt werden. Dieses wirkt mit höherer Kraft auf die zu zerstäubenden Partikel ein, so dass sich der Feinanteil des Aerosols erhöht.
Exemplarisch wurde eine solche Messung auch mit einem Düsenkanal (3a) mit ovalem statt rundem Querschnitt durchgeführt.
Bei gleicher Querschnittsfläche ergab sich bei Messungen mit dem ovalen Düsenkanal ein vergleichbarer FPD-Wert wie beim runden Düsenkanal, die Entleerung der Pulverkavität war bei Verwendung des ovalen Kanals erhöht. Weitere Messungen (ohne Treibmittelpulsung) mit unterschiedlichen Düsenkanälen (3a) zeigen neben der besseren Deagglomeration von Partikeln bei kleinen Querschnitten der Düsenkanäle (3a) jedoch einen weiteren Effekt auf: Die insgesamt aus dem Zerstäuber ausgebrachte Formulierungsmenge zeigt eine eigene Abhängigkeit vom Querschnitt des Düsenkanals (3a). Bei einer Querschnittsfläche von 0,2 Quadratmillimeter zeigt sie den im Vergleich niedrigsten Wert (in der konkreten Messreihe 62% bezogen auf die in der Pulverkavität (1) eingefüllte Formulierungsmenge), bereits bei 0,4 Quadratmillimeter wird ein deutlich höherer Wert (73%) erreicht, der zu wiederum höheren Querschnitten zunächst noch leicht zunimmt (76% bei 0,5 Quadratmillimetern) und anschließend tendenziell wieder leicht abnimmt (73% bei 0,8 Quadratmillimetern). Bei der Verwendung von Düsen (3) mit sehr engen Düsenkanälen (3a) wird weniger Pulver aus dem System ausgebracht, und stattdessen bleibt mehr Pulver in der Pulverkavität (1) zurück.

Um auch bei kleinen Durchmessern für den Düsenkanal (3a) dennoch die Entleerung zu begünstigen, ist dem Düsenkanal (3a) ein Einlasskonus (3b) vorgeschaltet, um den Eintritt für das Pulver zu erleichtern. Da in dem Düsenkanal (3a) das Aerosol eine vielfach höhere Geschwindigkeit als die angelegte Flussrate im Mundstück (2) erreicht, befindet sich zusätzlich noch ein Auslasskonus (3c) am Ende der Düse (3), um auftretende Turbulenzen beim Austritt zu reduzieren. Exemplarische Messungen deuten des Weiteren darauf hin, dass sich die insgesamt aus dem Zerstäuber ausgebrachte Formulierungsmenge durch die Verwendung von ovalen Querschnitten erhöht. Umso mehr konnte dem Effekt, dass die Entleerung aus der Pulverkavität (1) beim Übergang zu den kleineren Düsenkanälen (3a) schlechter wird, jedoch durch die Pulsung des Treibmittels entgegengewirkt werden. Insbesondere bei Durchmessern des Düsenkanals (3a) von lediglich 0,5 Millimetern konnten bei Verwendung von gepulsten Treibmittelstößen gute Ausbringungen erzielt werden (Beispiel 92,5% Ausbringung von 75 Milligramm einer Pulvermischung mit 98% Fenoterol aus wannenförmiger Pulverkavität (1) unter Erlangung von 16,5 Milligramm Fenotorol als tatsächlichen Feinanteil). Durch die Aufteilung eines Treibmittelstoßes in mehrere kleine Schübe oder Pulse, kann man so einerseits mit jedem Puls einen hohen inhalierbaren Feinanteil erhalten (unter Ausnutzung kleiner Querschnitte für den Düsenkanal (3a)) als auch andererseits durch die summierte Einwirkung der Pulse eine effiziente Entleerung der Pulverkavität erreichen. Dies gilt bereits für Pulverkavitäten (1) mit wannenförmigem Napf (1a) wie sie in Fig. 7a zu sehen sind, ist aber auch auf tropfenförmige Pulverkavitäten (1) wie sie in Fig. 7b zu sehen sind, und auf andere Kavitätenformen anwendbar, insbesondere wenn diese für die Aufnahme großer Pulvermengen im Bereich größer 21 Milligramm ausgelegt sind und die große Pulvermenge nicht direkt mit einem Treibgasstoß durch eine Düse kleinen Durchmessers ausgetrieben werden kann.

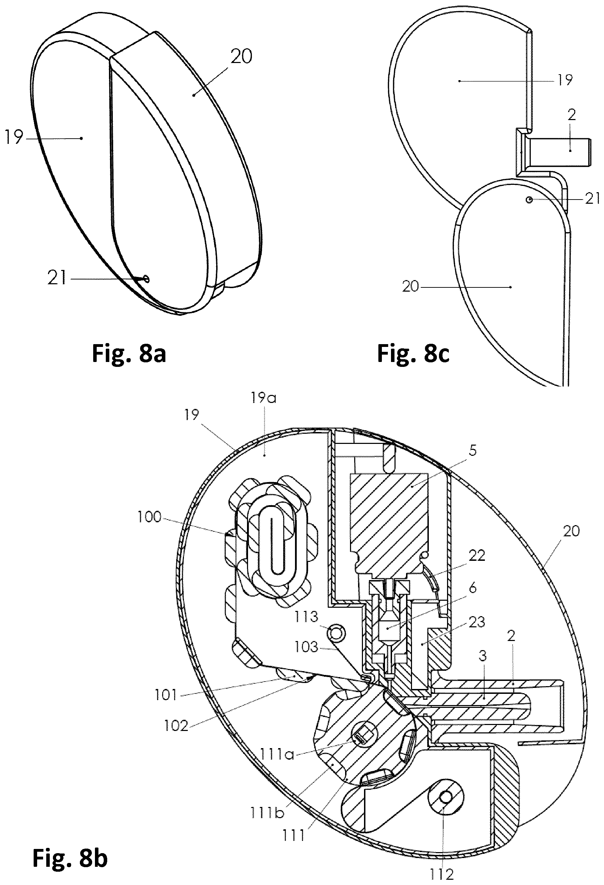

Fig. 8 zeigt einen erfindungsgemäßen Zerstäuber, der als Inhalator von einem Patienten angewendet werden kann und als kompaktes Handgerät ausgelegt ist. Die Funktionsweise dieses Inhalators ist die gleiche wie die anhand der Testvorrichtung aus Fig. 2 beschriebene. Insbesondere sind alle vorangegangenen Schilderungen, die das Mundstück (2). die Düse (3), die Kartusche (5) und den Verdampfer (6) betreffen, ebenfalls auf den in Fig. 8 gezeigten Inhalator anzuwenden. Wie Fig. 8 zu entnehmen ist sind diese Komponenten ganz analog wie im Ausführungsbeispiel gemäß Fig. 2 angeordnet. Fig. 8a zeigt das Äußere des hier ausgeführten Inhalators mit einer geschlossenen Mundstückabdeckung, Fig. 8c das Äußere mit einer geöffneten Mundstückabdeckung. Der Inhalator weist als besagte Mundstückabdeckung eine Abdeckung (20) auf, die über eine Drehachse (21) mit einem Gehäuse (19) verbunden ist. Wenn der Inhalator nicht benutzt wird, kann die Abdeckung (20) geschlossen werden und verdeckt das Mundstück (2) des Inhalators. In diesem Transportzustand mit geschlossener Abdeckung (20) sind nur Gehäuse (19) und Abdeckung (20) von außen zugänglich, und alle für die Zerstäubung funktionsrelevanten Bauteile sind gegen Verschmutzungen geschützt. Die Anbindung der Abdeckung (19) über eine hier pivotal ausgeführte Drehachse (21) ermöglicht ein einfaches Öffnen des Inhalators und stellt sicher, dass die Abdeckung (20) am Inhalator verbleibt und nicht verloren gehen kann.
Fig. 8b zeigt einen schematischen Schnitt des Inhalators, der in diesem Ausführungsbeispiel als pmDPI ausgelegt ist, d.h. als Mehrdosis-Gerät mit einzelnen vorabgemessenen und einzeln bevorrateten Dosiseinheiten einer pulverförmigen Formulierung. Die einzelnen Dosiseinheiten sind hier bevorzugt auf streifenförmigen Trägern angeordnet, insbesondere einem sogenannten Blisterband (100), das hintereinander gereihte Blisterkavitäten (101) aufweist, die zwischen einer Trägerbahn (102) und einer Deckfolie (103) gebildet werden. Insbesondere weist die bevorzugt aus einem Kunststoff und/oder Aluminium gefertigte Trägerbahn hierzu Vertiefungen auf, die z.B. in einem Tiefziehprozess gefertigt wurden. Vorzugsweise weisen die Blisterkavitäten (101) wannenförmige oder tropfenförmige Näpfe (1a) auf, wie sie anhand von Fig. 7 a-c beschrieben wurden. Die Blisterkavitäten (101) sind mit der pulverförmigen Formulierung gefüllt und können schrittweise mittels eines Rads (111) an eine Position gebracht werden, in der sie jeweils die Funktion der Pulverkavität (1) in einer funktionalen Anordnung analog zu der Testvorrichtung in den vorangegangenen Figuren übernehmen. Das Rad (111) weist hierzu entlang seines äußeren Umfangs äquidistant verteilt napf- oder taschenförmige Aufnahmen (111b) auf, welche die Blisterkavitäten (101) auf der Seite ihrer Trägerbahn (102) aufnehmen können und jeweils in die Pulverentnahmeposition mit Anschluss an die Treibmittelzuleitung aus dem Verdampfer (6) und an die Düse (3) drehen. An der Pulverentnahmeposition weist das Gerät eine Abdichtung auf, die verhindert, das Treibgas an der Blisterkavität (101) vorbei ausweichen kann und nur durch die Düse wieder austritt. Bevorzugt wird vor Erreichen der Pulverentnahmeposition die Deckfolie (103) von der Trägerbahn abgezogen und so geöffnet. Die Deckfolie wird vorzugsweise auf einer Spule (113) aufgewickelt. Bezogen auf den Vortrieb des Blisterbands (100) wird die Deckfolie (103) erst so spät von der Trägerbahn (102) separiert, dass immer nur die Blisterkavität (101), die zur gerade Pulverentnahmeposition gebracht wird, geöffnet ist und keine weiteren Blisterkavitäten (101), die noch Pulver enthalten, geöffnet werden. Die Trägerbahn (102) mit den entleerten Vertiefungen der Blisterkavitäten (101) wird auf einer weiteren Spule (112) aufgewickelt. Hierzu wird sie bevorzugt zwischen Pulverentnahmeposition und Spule (112) durch eine, nicht in der Abbildung gezeigte, Vorrichtung geführt, in der die Trägerbahn (102) geglättet und/oder die in ihr enthaltenen Vertiefungen platt gedrückt werden. Eine solche Vorrichtung ist z.B. der Schrift WO2007096111A2 (Seite 5 und den Ausführungen zu den dortigen Figuren 2 und 4) zu entnehmen.

An der Pulverentnahmeposition wird die geöffnete Blisterkavität (101) dicht an das Anschlussstück, das die Treibmittelzuführung und den Einlass des Düsenkanals (3a) aufweist, herangeführt oder angedrückt. Beispielsweise ist das Rad (111), das Blisterband (100) an dieser Stelle so orientiert, dass die Blisterkavität (101) gegen das Anschlussstück gedrückt oder gezwängt wird. Die Aufnahmen (111b) am Rad (111) sind dabei derart gestaltet, dass die Oberseite der Blisterkavität (101) die gleiche Wölbung wie die zugehörige Unterseite des Anschlussstücks aufweist - bevorzugt liegt die Blisterkavität (101) komplett flach auf dem Rad (111) auf und ist nicht gewölbt. Bevorzugt beinhaltet das Anschlussstück Materialien, die insbesondere den oberen äußeren Rand der geöffneten Blisterkavität (101) gegen das Anschlussstück abdichten, wie eine Teflonbeschichtung oder einen in die Kontaktoberfläche eingelassenen Dichtungsring.
Zusätzlich ist die Drehachse (111a) des Rads (111) bevorzugt derart durch eine Feder oder ähnliches mit Druck beaufschlagt, so dass das Rad (111) in Richtung des Anschlussstücks gedrückt und auf diese Weise die Dichtigkeit gewährleistet wird.
Alternativ zum Rad (111) kann eine vorzugsweise federvorgespannte Führungsschiene verwendet werden. Über eine Zugkraft an der Spule (112) wird dann zunächst die Blisterkavität (101) in ihre Position am Anschlussstück gebracht und anschließend durch diese Führungsschiene angedrückt. Je nach Gestaltung des Blistersbandes (100) kann die Führungsschiene dabei eine glatte Oberfläche haben (dann eignet sie sich auch zur Führung des Blisterbandes beim Weitertransport im Gerät) oder eine bewegliche Druckplatte mit Aufnahme sein, wobei diese Platte während des Transport des Blisterbandes (100) dieses nicht berührt.
In einer Ausführungsform des erfindungsgemäßen Zerstäubers entspricht der Mechanismus zum Abziehen der Deckfolie (103) vorzugsweise dem entsprechenden Mechanismus, der in der Schrift DE4106379A1 offenbart wird. Eine Weiterentwicklung dieses Transportmechanismus findet sich in der EP1436216B1.
Bevorzugt wird der Vortrieb des Blisterbandes (100) durch eine Bewegung der Abdeckung (20), vorzugsweise durch die Öffnungsbewegung, bewirkt. Hierzu ist die Drehachse (21) vorzugsweise an die Spule (112) gekoppelt, so dass beim Öffnen des Geräts das Blisterband (100) in Vortriebsrichtung gezogen wird und sich das Rad (111) dabei mit dreht. Bei dieser Kopplung wird dann eine Rücklaufsperre beispielsweise in Form einer Rutschkupplung in der Art vorgesehen, dass die Drehbewegung der Achse (21) nur in einer Richtung (vorzugsweise der Öffnungsrichtung) auf die Spule (112) übertragen wird. Zusätzlich wird optional die Drehachse (21) und /oder Spule (112) über ein, in Fig. 8 nicht dargestelltes, Getriebe mit der Achse (111a) des Rads (111) und/oder der Spule (113) zum Aufwickeln der Deckfolie (103) gekoppelt. Hierbei besteht auch die Möglichkeit, dass die Drehachse (21) direkt auf die Achse (111a) des Rads (111) wirkt und dies über ein Getriebe seinerseits mit den beiden Spulen (112, 113) verbunden ist. Bezüglich des möglichen Aufbaus eines solchen Getriebes und der Rücklaufsperre sei hier auf die Schrift WO2007134792A1 (Seite 4 Zeile 30-34, Seite 6 Zeile 30 bis Seite 7 Zeile 13, Seite 8 Zeile 7 bis 29, Seite 9 Zeile 21 bis 29 und Seite 10 Zeile 25 bis Seite 14 Zeile 13) verwiesen.
Die Rücklaufsperre bezüglich der Übertragung der Drehbewegung der Drehachse (21) und der Achse (111a) des Rads (111) und/oder der Spulen (112, 113) kann des Weiteren entsprechend der Rücklaufsperre gestaltet werden, die in der Schrift WO07068896 offenbart wird.

### Alternative elektrische Ansteuerung Vortrieb

Alternativ zum Abziehen der Deckfolie (103) und der Verwendung einer zugehörige Spule (113) können die Blisterkavitäten (101) auch vor Erreichen der Pulverentnahmeposition an einer anders gearteten Öffnungsvorrichtung vorbei geführt werden, an der die Deckfolie (103) an der Stelle der Blisterkavität (101) beispielsweise angestochen oder aufgeschnitten oder anderweitig geöffnet wird. Bevorzugt ist der Inhalator so gestaltet, dass sich Blisterband (100), Rad (111) und die Spulen (112, 113) und gegebenenfalls zwischen diesen wirkende Getriebe-Elemente in einem austauschbaren Gehäuseteil (19a) befinden. Auf diese Weise wird die Größe des Inhalators nicht durch die Länge des Blisterbandes (100), d.h. nicht durch die maximal mögliche Anzahl von Dosierungen bestimmt.

Zur Ausbringung des Pulvers aus der in die Pulverentnahmeposition gebrachten Blisterkavität (101) wird Treibmittel aus der Kartusche (5) abgegeben. Dies kann entweder dadurch geschehen, dass der Anwender bzw. Patient direkt auf die Kartusche (5) in Richtung ihres in dem Fall bevorzugt gefedert gelagertenVentilstammes (7) drückt (Druckausübung auf Kartuschen-Boden auf der dem Ventilstamm (7) entgegengesetzten Seite) oder dadurch, dass der Patient durch Einatmen am Mundstück (2) eine entsprechende Bewegung der Kartusche (5) und/oder die Pulssequenz an einem eingebauten Magnetventil auslöst.

Eine solche so genannte Atemzugstriggerung ist in dem in Fig. 5a skizzierten Gerät vorgesehen.
Fig. 5 zeigt eine verkippbare Fahne (22), welche die Kartusche (5) in einer Ruheposition leicht von der Ventilstammaufnahme (8a) beabstandet, so dass das Ventil der Kartusche (5) geschlossen ist. Atmet der Patient nun am Mundstück (2) ein, so erzeugt er insbesondere an der Einlassöffnung (2b) der Mundstücks einen Sog, der sich ebenfalls auf den daran angeschlossenen Hohlraum erstreckt, der im Ausführungsbeispiel einen Bypass (23) darstellt, in dem Luft neben dem Verdampfer vorbei strömen kann. Die mit der Umgebungsluft verbundene Einlassöffnung dieses Bypasses ist in Ruheposition durch einen Teil der Fahne (22) verschlossen. Durch den Sog beim Einatemnvorgang kippt die Fahne (22) derart, dass an ihrem einen Ende die Einlassöffnung am Bypass (23) freigegeben wird. Dadurch wird eine Blockierung der Kartusche (5) gelöst und der Bewegungsweg für die Kartusche (5) am anderen Ende der Fahne (22) wird nach unten bzw. in Richtung der Ventilstammaufnahme (8a) freigegeben. Bevorzugt ist die Kartusche (5) derart federvorgespannt gelagert, dass sie sich selbst bei Auslenkung der Fahne (22) beim Einatemvorgang in Richtung der Ventilstammaufnahme (8a) bewegt. Lässt der durch das Einatmen am Mundstück (2) erzeugte Sog im Bypass (23) nach, stellt sich die Fahne (22) in ihre ursprüngliche Position zurück und die Kartusche (5) wird wieder von der Ventilstammaufnahme (8a) beabstandet. Je nach Stärke der Atemzugstriggerung kann die Fahne (22) hierzu auch mit einem Rückstellmechanismus verbunden sein. Nach der Atemzugsauslösung der Bewegung der Kartusche (5) wird die Kartusche vorzugsweise beim Schließen oder Öffnen der Abdeckung (20) - vorzugsweise über eine Kulissenführung - wieder in die vorgespannte Ausgangssituation gebracht.
Eine solche Kombination aus Vorspannung einer Treibmittel-Kartusche und einer Atemzugstriggerung wird in der US5031610 offenbart. In der US5031610 wird die Vorspannung der Kartusche und die Vorbereitung der Atemzugstriggerung durch Absetzen und erneutes Aufstecken einer Kappe auf dem Mundstück verursacht. In einer hier bevorzugten Ausführungsform würde der Mechanismus aus der US5031610 statt dessen an die Schwenkbewegung der Abdeckung (20) oder an einen zusätzlichen, nicht gezeigten Hebel gekoppelt werden.

Alternativ zu so einer mechanischen Atemzugstriggerung kann auch eine elektromechanische Steuerung verwendet werden. In solchen (nicht gezeigten) Ausführungsformen mit elektromagnetischer Steuerung weist der Zerstäuber vorzugsweise eine Batterie auf, welche die für solche Steuerungen benötigten elektrischen Spannungen zur Verfügung stellt. Bei einer solchen elektrischen oder elektromechanischen Atemzugstriggerung weist der Zerstäuber innen am Mundstück (2) einen elektrischen Flusssensor auf, der in Abhängigkeit von der detektierten Strömung ein mit der Flussrate variierendes elektrisches Signal abgibt. Dieses Signal wird dann verwendet um einen elektromechanischen Vorgang zu starten, durch den z.B. die Kartusche (5) in Richtung der Ventilstammaufnahme (8a) bewegt wird, das Ventil der Kartusche (5) geöffnet und somit Treibmittel in den Verdampfer (6) bzw. die Kanäle des Zerstäubers freigesetzt wird. Damit diese Ventilbetätigung erst bei einer vordefinierten Luftströmung, d.h. einem bestimmten Sog am Mundstück (2), stattfindet wird das Sensor-Signal zuvor durch eine Kontrollvorrichtung beispielsweise eine analoge Vergleichsschaltung oder eine digitale Elektronik geleitet. Das Sensor-Signal löst gewissermaßen bei Erreichen eines bestimmten Sogs am Mundstück (2) einen elektrischen Schalter aus. Bei Betätigung dieses elektrischen Schalters wird ein elektromechanischer Vorgang gestartet, beispielsweise ein Schrittmotor in Gang gesetzt, der die Kartusche verschiebt. Solche elektromechanischen Atemzugstriggerungen von Zerstäubern mit Treibmittel-Kartuschen sind in der Schrift WO9207599A1 offenbart.

Eine von der Bewegung der Kartusche unabhängige Möglichkeit eine Atemzugstriggerung im Zerstäuber einzubringen besteht darin (bevorzugt für den Fall, dass kein durch einen elektronischen Pulsgeber angesteuertes Magnetventil wie in Fig. 4 verwendet werden soll), dass zusätzlich zu dem zur Kartusche (5) gehörenden Ventil ein zweites Ventil vorgesehen wird. Das zweite Ventil befindet sich bei einer solchen Ausführungsform strömungstechnisch gesehen vor dem Einlass des Verdampfers (6). Der Zerstäuber wird für die Inhalation derart vorbereitet, dass das 1. Ventil betätigt wird - z.B. durch eine Verschiebung der Kartusche (5), wobei die Verschiebung an das Öffnen der Abdeckung (20) gekoppelt ist - und das abgegebene Treibmittel fließt in eine Vorkammer vor dem zweiten Ventil. Bei Auslösung der Atemzugstriggerung wird dann lediglich das 2. Ventil geöffnet, was eine geringere Krafteinleitung benötigt als die für die Auslösung des ersten Ventils benötigte Verschiebung der Kartusche (5). Eine solche Ausführungsform mit einem zweiten Ventil zusätzlich zu dem Dosierventil einer Kartusche (5) kann eine Ventilanordnung und deren Kopplung mit einer Atemzugssteuerung aufweisen, wie sie in der Schrift GB2233236A offenbart wird, die auf die Atemzugstriggerung eines MDI gerichtet ist. Das zweite Ventil wird direkt durch den Sog des Patienten geöffnet, z.B. durch das Ansaugen eines Tellers oder Stempels, der mit der Öffnung des zweiten Ventils verbunden ist oder durch das indirekten Ansaugen einer Komponente, die zu einem ansonsten durch magnetische Kräfte verschlossenen zweiten Ventil gehört. Ein solcher Teller oder Stempel wird in der hier betrachteten Ausführungsform des Zerstäubers bevorzugt im Bypass (23) vorgesehen. Bevorzugt ist das zweite Ventil dabei zusätzlich z.B. durch einen federnden Anschlag bei Auslenkung so beschaffen und/oder mittels zusätzlicher Vorspannung so eingestellt, dass es bei Auslösung durch den Atemzug pulsartig öffnet, d.h. nach Öffnung in die Schließstellung zurückfedert, im anhaltenden Atemzug wieder öffnet, wieder schließt und so weiter.
Bevorzugt werden die hier beschriebenen Zerstäuber mit einer medizinischen Formulierungen betrieben, die ein Bestandteil aus der Offenbarung der europäischen Patentanmeldung mit der Anmeldenummer 12151105.9 auf Seite 26 Zeile 12 bis Seite 63 Zeile 2 aufweist oder einer der dort genannten Formulierungen entspricht.

### Bezugszeichenliste

- 1: Pulverkavität
- 1a: Napf (in Pulverkavität)
- 1b: Dichtungsnut
- 1c: Schräge (in Pulverkavität)
- 1d: Abflachung (in Pulverkavität)
- 1t: Träger (von Pulverkavität)
- 2: Mundstück
- 2a: Durchführung (am Mundstück)
- 2b: Einlassöffnung (am Mundstück)
- 2l: Länge (des Mundstücks)
- 3: Düse
- 3a: Düsenkanal
- 3b: Einlasskonus (der Düse)
- 3c: Auslasskonus (der Düse)
- 3d: Endfläche (der Düse)
- 3g: Anschlussseite (der Düse)
- 3f: Sicke
- 3l: Länge (des zylindrischen Teils des Düsenkanals)
- 4: Anschlussstück
- 5: Kartusche
- 6: Verdampfer
- 7: Ventilstamm
- 8: Deckel (am Verdampfer)
- 8a: Ventilstammaufnahme (im Deckel)
- 8b: Flansch (am Deckel)
- 8c: Sicke (im Flansch)
- 8d: Innenkonus (am Deckel)
- 9: Körper (des Verdampfers)
- 9a: Hohlraum (des Verdampfers)
- 9b: Trichter
- 9c: Auslass (des Verdampfers)
- 9d: Stamm (am Verdampfer)
- 10: Dichtung
- 11: Kugel
- 12: Draht
- 13: Magnetventil
- 14: Flanschplatte
- 15: mikrofluidischer Oszillator
- 15a: Einlasskanal (am mikrofluidischen Oszillator)
- 15b: Teilkanäle
- 15c: Innere Wände (der Teilkanäle)
- 15d: Mischbereich
- 15e: Vorsprünge im Mischbereich
- 15f: Auslass (aus Mischbereich des mikrofluidischen Oszillators)
- 15t: Strömungsteiler
- 15v: Lüftungskanal
- 19: Gehäuse
- 19a: austauschbares Gehäuseteil
- 20: Abdeckung (für Mundstück)
- 21: Drehachse (für Abdeckung)
- 22: Fahne
- 23: Bypass
- 100: Blisterband
- 101: Blisterkavität
- 102: Trägerbahn
- 103: Deckfolie
- 111: Rad
- 111a: Drechachse (an Rad)
- 111b: Aufnahme (an Rad)
- 112: Spule (für Trägerbahn)
- 113: Spule (für Deckfolie)

- α: Einlasswinkel (an Düse)
- β: Auslasswinkel (an Düse)

- A: Aerosol
- G: Pulsgenerator
- I: Zerstäuber
- K: Treibgaskartusche
- Q: Quelle (Gas)
- R(p): Druckregler
- R(F): Flussregler
- V: Magnetventil
- WT: Wärmetauscher

## Patentansprüche

1. Gerät zur Zerstäubung einer pulverförmigen medizinischen Formulierung, bei dem die Zerstäubung durch ein Treibmittel unterstützt wird, wobei sich eine abgemessene Menge der Formulierung in einer Pulverkavität (1) befindet, und wobei das Gerät einen Behälter oder eine Kartusche (5), in der das Treibmittel in Form von verflüssigtem Treibgas bevorratet ist, und einen Zuführungskanal aufweist, mittels dem das Treibmittel der Pulverkavität (1) zugeführt werden kann, so dass das Treibmittel die Formulierung aus der Pulverkavität (1) durch eine Düse (3) austreibt, wobei das Gerät ein Dosierventil zur Entnahme eines abgemessenen Volumens an flüssigem Treibmittel aus dem Behälter oder der Kartusche (5) aufweist und sich stromabwärts des Dosierventils eine Vorrichtung zur Erzeugung von Pulsen im Treibmittel befindet, die einen Einlass hat, an dem das Treibmittel anliegt, und durch die das Treibmittel durchgeleitet werden kann, wobei die Vorrichtung derart konstruiert ist, dass sie bei Durchleitung des Treibmittels in diesem ein derartiges Strömungsverhalten bewirkt, dass das abgemessenen Treibmittel-Volumen aus der Vorrichtung in Form von mehreren aufeinander folgenden, von einander abgegrenzten Pulsen oder Schüben austritt und in Form von mehreren aufeinander folgenden, von einander abgegrenzten Pulsen oder Schüben der Pulverkavität (1) zugeführt wird und das Pulver aus der Pulverkavität (1) mittels mehrerer Treibmittelschübe oder Treibmittelpulse in einem Zeitintervall ausgebracht wird, das in seiner Dauer einem Einatemzug eines Patienten entspricht, dadurch charakterisiert, dass die jeweils mehreren aufeinander folgenden Pulse oder Schübe in derart von einander abgegrenzt sind, dass die Strömung des Treibmittels zwischen den jeweiligen Pulsen oder Schüben abreißt.

2. Gerät nach Anspruch 1, **dadurch gekennzeichnet, dass** die Vorrichtung die Pulse im Treibmittel derart erzeugen kann, dass das abgemessene Volumen an Treibmittel derart in Pulse oder Schübe aufgeteilt wird, dass die Aufteilung des abgemessenen Volumens in Pulse oder Schübe einer Aufteilung von 100 Mikrolitern auf mindestens 7 Pulse oder Schübe entspricht.

3. Gerät nach einem der vorherigen Ansprüche **dadurch gekennzeichnet, dass** die Vorrichtung zur Erzeugung von Pulsen im Treibmittel ein Ventil, insbesondere ein ansteuerbares Magnetventil (13), beinhaltet, das durch mehrere Öffnungs- und Schließvorgänge das anliegende Treibmittel in mehrere Pulse aufteilt.

4. Gerät nach Anspruch 3 **dadurch gekennzeichnet, dass** das Ventil während des Betriebs des Geräts Öffnungszeiten im Bereich von 3 bis 30, besonders bevorzugt im Bereich von 5 bis 10 Millisekunden und Verschlusszeiten im Bereich von 60 bis 500 Millisekunden, besonders bevorzugt im Bereich von 100 bis 200 Millisekunden aufweist.

5. Gerät nach Anspruch 1 **dadurch gekennzeichnet, dass** das Treibmittel über eine einen mikrofluidischen Oszillator bildende Kanalstruktur zugeführt wird, in der die Pulse oder Schübe im Treibmittel erzeugt werden, wobei die Kanalstruktur mindestens eine Gabelung und einen Mischbereich (15d) für Fluidstrahlen aufweist.

6. Gerät nach einem der vorangegangenen Ansprüche **dadurch gekennzeichnet, dass** das Treibmittel vor seiner Zuführung in die Pulverkavität (1) durch einen Verdampfer (6) oder Wärmetauscher geleitet wird.

7. Gerät nach Anspruch 6 **dadurch gekennzeichnet, dass** der Verdampfer (6) einen Hohlraum (9a) aufweist und in seinem Hohlraum (9a) ein oder mehrere Wärmetausch-Elemente beinhaltet, die insbesondere durch Metallkugeln und/oder Metalldrähte gebildet werden.

8. Gerät nach einem der vorangegangenen Ansprüche **dadurch gekennzeichnet, dass** ein mit Formulierung beladener Treibmittelstrom von der Pulverkavität (1) aus in eine Düse (3) geleitet wird und die Düse (3) einen im Wesentlichen geradlinigen Düsenkanal (3a) aufweist.

9. Gerät nach Anspruch 8 dadurch charakterisiert, dass die Zuleitung von Treibmittel in die Pulverkavität (1) und die Achse durch den Düsenkanal (3a) beide in einem Winkel zwischen 30° und insbesondere 45° in der Pulverkavität (1) und/oder in diesem Winkel relativ zum Boden der Pulverkavität (1) eintreffen, wobei beide Winkel vorzugsweise gleich groß sind.

10. Gerät nach einem der Ansprüche 8 oder 9 **dadurch gekennzeichnet, dass** der Düsenkanal (3a) einen Einlasskonus (3b) und/oder einen Auslasskonus (3c) aufweist und/oder die Wand des Düsenkanals (3a) zumindestens in einem zentralen Bereich zylinderförmig ist.

11. Gerät nach einem der Ansprüche 8 bis 10 **dadurch gekennzeichnet, dass** die Pulverkavität (1), in der sich eine abgemessene Menge der pulverförmigen Formulierung befindet stromlinienförmig ist.

12. Gerät nach Anspruch 11 **dadurch gekennzeichnet, dass** die Pulverkavität (1) eine Tropfenform aufweist, wobei das schmalere Ende des Tropfens in Richtung des Einlass des Düsenkanals (3a) zeigt.

13. Gerät nach einem der Ansprüche 11 oder 12 **dadurch gekennzeichnet, dass** die Pulverkavität (1) eine Schräge (1c) am Boden der Pulverkavität (1) aufweist, wobei diese Schräge (1c) die Strömung in Richtung des Einlass des Düsenkanals (3a) leitet, wobei vorzugsweise die Richtung der Schräge (1c) und des Düsenkanals (3a) identisch sind.

14. Verfahren zur Zerstäubung von pulverförmigen medizinischen Formulierungen mittels eines Geräts, bei dem die Zerstäubung durch ein Treibmittel unterstützt wird, das in flüssiger Form mittels eines Dosierventils abgemessen wird und anschließend in mehrere Schübe aufgeteilt, so dass das Treibmittel in Form von mehreren aufeinander folgenden, von einander abgegrenzten Pulsen oder Schüben mittels eines Zuführungskanals einer Pulverkavität (1) zugeführt wird, in der sich eine abgemessene Menge der Formulierung befindet, wobei das Pulver aus der Pulverkavität (1) mittels der mehreren Treibmittelpulse oder Treibmittelschübe in einem Zeitintervall ausgebracht wird, das in seiner Dauer einem Einatemzug eines Patienten entspricht, dadurch charakterisiert, dass die jeweils mehreren aufeinander folgenden Pulse oder Schübe in derart von einander abgegrenzt sind, dass die Strömung des Treibmittels zwischen den jeweiligen Pulsen oder Schüben abreißt.

## Claims

1. Apparatus for nebulising a powdered medicinal formulation, wherein the nebulisation is assisted by a propellant, a measured amount of the formulation being contained in a powder cavity (1), and the apparatus comprising a container or a cartridge (5) in which the propellant is stored in the form of liquified propellant gas, and comprising a feed channel by means of which the propellant can be fed to the powder cavity (1) such that the propellant expels the formulation out of the powder cavity (1) through a nozzle (3), the apparatus having a metering valve for removing a measured volume of liquid propellant from the container or the cartridge (5), and a device for generating pulses in the propellant being located downstream of the metering valve, which device has an inlet at which the propellant is present, and through which the propellant can be conveyed, the device being designed in such a way that it causes such flow characteristics in the propellant as said propellant passes through that the measured propellant volume exits the device in the form of a plurality of successive pulses or bursts which are kept distinct from one another and is supplied to the powder cavity (1) in the form of a plurality of successive pulses or bursts which are kept distinct from one another, and the powder is discharged from the powder cavity (1) by means of a plurality of propellant bursts or propellant pulses in a time interval of which the duration corresponds to an inhalation of a patient, **characterised in that** the plurality of respective successive pulses or bursts are kept distinct from one another such that the flow of propellant comes to a standstill between the respective pulses or bursts.

2. Apparatus according to claim 1, **characterised in that** the device is able to generate the pulses in the propellant such that the measured volume of propellant is divided into pulses or bursts in such a way that the division of the measured volume into pulses or bursts corresponds to a distribution of 100 microlitres between at least 7 pulses or bursts.

3. Apparatus according to one of the preceding claims, **characterised in that** the device for generating pulses in the propellant contains a valve, in particular an actuatable magnetic valve (13), which divides the propellant present into a plurality of pulses by means of a plurality of opening and closing processes.

4. Apparatus according to claim 3, **characterised in that** during the operation of the apparatus the valve has opening times in the range from 3 to 30, particularly preferably in the range from 5 to 10 milliseconds, and closure times in the range from 60 to 500 milliseconds, particularly preferably in the range from 100 to 200 milliseconds.

5. Apparatus according to claim 1, **characterised in that** the propellant is supplied through a channel structure forming a microfluidic oscillator in which the pulses or bursts are produced in the propellant, the channel structure comprising at least one fork and a mixing region (15d) for fluid jets.

6. Apparatus according to one of the preceding claims, **characterised in that** before being fed into the powder cavity (1) the propellant is passed through a vaporiser (6) or heat exchanger.

7. Apparatus according to claim 6, **characterised in that** the vaporiser (6) has a cavity (9a) and contains in its cavity (9a) one or more heat exchange elements which are formed in particular by metal beads and/or metal wires.

8. Apparatus according to one of the preceding claims, **characterised in that** a propellant stream charged with formulation is conveyed from the powder cavity (1) into a nozzle (3) and the nozzle (3) comprises a substantially rectilinear nozzle channel (3a).

9. Apparatus according to claim 8, **characterised in that** the feed line for propellant into the powder cavity (1) and the axis through the nozzle channel (3a) both meet at an angle of between 30° and particularly 45° in the powder cavity (1) and/or at this angle relative to the base of the powder cavity (1), the two angles preferably both being the same.

10. Apparatus according to one of claims 8 or 9, **characterised in that** the nozzle channel (3a) comprises an inlet cone (3b) and/or an outlet cone (3c) and/or the wall of the nozzle channel (3a) is cylindrical, at least in a central region.

11. Apparatus according to one of claims 8 to 10, **characterised in that** the powder cavity (1) in which a measured amount of the powdered formulation is contained is streamlined in shape.

12. Apparatus according to claim 11, **characterised in that** the powder cavity (1) has a teardrop shape, the narrower end of the teardrop pointing in the direction of the inlet of the nozzle channel (3a).

13. Apparatus according to one of claims 11 or 12, **characterised in that** the powder cavity (1) has a slope (1c) on the bottom of the powder cavity (1), this slope (1c) conveying the flow in the direction of the inlet of the nozzle channel (3a), while preferably the direction of the slope (1c) and of the nozzle channel (3a) are identical.

14. Method for nebulising powdered medicinal formulations by means of an apparatus, wherein the nebulisation is assisted by a propellant which is measured in liquid form by means of a metering valve and subsequently divided into a plurality of bursts, such that the propellant is supplied, in the form of a plurality of successive pulses or bursts distinct from one another, by means of a feed channel to a powder cavity (1) in which there is a measured amount of the formulation, the powder being discharged from the powder cavity (1) by means of the plurality of propellant pulses or propellant bursts in a time interval of which the duration corresponds to an inhalation of a patient, **characterised in that** the plurality of respective successive pulses or bursts are kept distinct from one another such that the flow of propellant comes to a standstill between the respective pulses or bursts.

## Revendications

1. Appareil pour l'atomisation d'une préparation médicale en poudre, dans lequel l'atomisation est assistée par un agent propulseur, dans lequel une quantité mesurée de la préparation se trouve dans une cavité à poudre (1), et dans lequel l'appareil présente un conteneur ou une cartouche (5) dans laquelle l'agent propulseur est stocké sous la forme d'un gaz propulseur liquéfié, et un canal d'acheminement, au moyen duquel l'agent propulseur peut être acheminé à la cavité à poudre (1) de sorte que l'agent propulseur fasse sortir la préparation de la cavité à poudre (1) par une buse (3),
dans lequel l'appareil présente une soupape de dosage pour le prélèvement d'un volume mesuré d'agent propulseur liquide du conteneur ou de la cartouche (5) et en aval de la soupape de dosage se trouve un dispositif pour la génération d'impulsions dans l'agent propulseur, qui a une entrée au niveau de laquelle l'agent propulseur s'applique, et au travers de laquelle l'agent propulseur peut être conduit, dans lequel le dispositif est construit de telle sorte que, lors du passage de l'agent propulseur dans celle-ci, un comportement d'écoulement est provoqué de telle sorte que le volume d'agent propulseur mesuré sorte du dispositif sous la forme de plusieurs impulsions ou poussées consécutives, séparées les unes des autres, et soit acheminé sous la forme de plusieurs impulsions ou poussées consécutives, séparées les unes des autres, à la cavité de poudre (1) et la poudre est évacuée de la cavité à poudre (1) au moyen de plusieurs poussées d'agent propulseur ou impulsions d'agent propulseur dans un intervalle temporel qui correspond dans sa durée à une respiration unique d'un patient,
**caractérisé en ce que**
les impulsions ou poussées respectivement consécutives sont séparées les unes des autres de telle sorte que l'écoulement de l'agent propulseur s'interrompe entre les impulsions ou poussées respectives.

2. Appareil selon la revendication 1, **caractérisé en ce que** le dispositif peut générer les impulsions dans l'agent propulseur de telle sorte que le volume mesuré d'agent propulseur soit réparti dans des impulsions ou poussées de telle sorte que la répartition du volume mesuré dans des impulsions ou poussées corresponde à une répartition de 100 microlitres d'au moins 7 impulsions ou poussées.

3. Appareil selon l'une quelconque des revendications précédentes, **caractérisé en ce que** le dispositif contient, pour la génération d'impulsions dans l'agent propulseur, une soupape, en particulier une soupape aimantée (13) pouvant être commandée qui répartit, par plusieurs processus d'ouverture et de fermeture, l'agent propulseur appliqué dans plusieurs impulsions.

4. Appareil selon la revendication 3, **caractérisé en ce que** la soupape présente, pendant l'actionnement de l'appareil, des temps d'ouverture dans la plage de 3 à 30, de manière particulièrement préférée dans la plage de 5 à 10 millisecondes et des temps de fermeture dans la plage de 60 à 500 millisecondes, de manière particulièrement préférée dans la plage de 100 à 200 millisecondes.

5. Appareil selon la revendication 1, **caractérisé en ce que** l'agent propulseur est acheminé par une structure canalaire formant un oscillateur microfluidique, dans laquelle les impulsions ou poussées sont générées dans l'agent propulseur, dans lequel la structure canalaire présente au moins une bifurcation et une zone de mélange (15d) pour des jets de fluide.

6. Appareil selon l'une quelconque des revendications précédentes, **caractérisé en ce que** l'agent propulseur est conduit, avant son acheminement dans la cavité à poudre (1), au travers d'un évaporateur (6) ou d'un échangeur de chaleur.

7. Appareil selon la revendication 6, **caractérisé en ce que** l'évaporateur (6) présente un espace creux (9a) et contient, dans son espace creux (9a), un ou plusieurs éléments échangeurs de chaleur, qui sont formés en particulier par des billes métalliques et/ou des fils métalliques.

8. Appareil selon l'une quelconque des revendications précédentes, **caractérisé en ce qu'**un courant d'agent propulseur chargé de la préparation est conduit de la cavité de poudre (1) dans une buse (3) et la buse (3) présente un canal de buse (3a) sensiblement rectiligne.

9. Appareil selon la revendication 8, **caractérisé en ce que** la conduite d'agent propulseur dans la cavité de poudre (1) et l'axe au travers du canal de buse (3a) se rencontrent tous les deux selon un angle entre 30° et en particulier 45° dans la cavité de poudre (1) et/ou selon cet angle par rapport au fond de la cavité à poudre (1), dans lequel les deux angles sont de préférence de même dimension.

10. Appareil selon l'une quelconque des revendications 8 ou 9, **caractérisé en ce que** le canal de buse (3a) présente un cône d'entrée (3b) et/ou un cône de sortie (3c) et/ou la paroi du canal de buse (3a) est de forme cylindrique au moins dans une zone centrale.

11. Appareil selon l'une quelconque des revendications 8 à 10, **caractérisé en ce que** la cavité à poudre (1), dans laquelle se trouve une quantité mesurée de préparation en poudre, est de forme aérodynamique.

12. Appareil selon la revendication 11, **caractérisé en ce que** la cavité à poudre (1) présente une forme de goutte, dans lequel l'extrémité plus étroite de la goutte est orientée en direction de l'entrée du canal de buse (3a).

13. Appareil selon l'une quelconque des revendications 11 ou 12, **caractérisé en ce que** la cavité à poudre (1) présente un biseau (1c) au fond de la cavité à poudre (1), dans lequel ce biseau (1c) conduit l'écoulement en direction de l'entrée du canal de buse (3a), dans lequel de préférence la direction du biseau (1c) et celle du canal de buse (3a) sont identiques.

14. Procédé pour l'atomisation de préparations médicales en poudre au moyen d'un appareil, dans lequel l'atomisation est assistée par un agent propulseur qui est mesuré sous forme liquide au moyen d'une soupape de dosage et ensuite est réparti en plusieurs poussées de sorte que l'agent propulseur soit acheminé sous la forme de plusieurs impulsions ou poussées consécutives, séparées les unes des autres, au moyen d'un canal d'acheminement d'une cavité à poudre (1), dans laquelle se trouve une quantité mesurée de la préparation, dans lequel la poudre sort de la cavité à poudre (1) au moyen des plusieurs impulsions d'agent propulseur ou poussées d'agent propulseur dans un intervalle temporel qui correspond dans sa durée à une respiration unique d'un patient,
**caractérisé en ce que**
les plusieurs impulsions ou poussées consécutives respectives sont séparées les unes des autres de telle sorte que l'écoulement de l'agent propulseur s'interrompe entre les impulsions ou poussées respectives.
